# FASCICULE DE BREVET EUROPEEN

(11) **EP 0 461 007 B1**
(45) Date de publication et mention de la délivrance du brevet: **08.11.1995**
(21) Numéro de dépôt: 91401406.3
(22) Date de dépôt: 30.05.1991
(51) Int. Cl.: A61F 5/448

(54) **Appareillage pour Stomie**
Stomavorrichtung
Stoma apparatus

(30) Priorité: 08.06.1990 FR 9007140; 14.03.1991 FR 9103091
(43) Date de publication de la demande: 11.12.1991
(73) Titulaire: B. BRAUN BIOTROL, 92100 Boulogne Billancourt (FR)
(72) Inventeur: Ozenne, Jean-Pierre, F-60580 Coye la Forêt (FR); Holtermann, Henri, F-64500 St Jean de Luz (FR)
(74) Mandataire: Orès, Bernard

(56) Documents cités:
- EP-A- 353 904
- EP-A- 0 171 255
- EP-A- 0 347 025
- WO-A-87/03192
- FR-A- 2 626 464

## Description

La présente invention est relative aux appareillages pour stomie et vise plus particulièrement, un dispositif d'assemblage d'un porte-poches et d'une poche de stomie.

De nombreux appareillages ont déjà été proposés pour le recueil des urines ou des déchets corporels chez des individus ayant subi une intervention chirurgicale comme une entérostomie (iléostomie, colostomie,...) ou une urostomie. On distingue, parmi ces appareillages connus, des dispositifs constitués par des poches à usage unique généralement fixées directement à la peau de l'utilisateur par un adhésif en forme d'anneau ou munies d'un protecteur cutané et des dispositifs "deux-pièces" dans lesquels la poche de recueil, -sous forme d'un sac jetable ou vidangeable-, peut être solidarisée de manière amovible sur une plaque frontale protectrice fixée au corps de l'utilisateur par un patin muni d'un adhésif ou d'une gomme adhésive ou par une ceinture (voir document EP-A-353904).

Des dispositifs du type de ceux mentionnés en dernier lieu doivent satisfaire à des conditions impératives dont certaines sont contradictoires entre elles. Il convient, en effet, que la fixation de la poche de recueil sur la plaque frontale protectrice ou porte-poches puisse se faire sans application d'une pression importante dans la zone voisine de la stomie, -qui est sensible et généralement douloureuse-, et que la fixation soit sûre avec une parfaite étanchéité de l'assemblage du système tout en permettant une séparation aisée mais jamais intempestive de la poche. Complémentairement, l'appareillage ne doit pas être à l'origine de complications cutanées péristomiales, doit être aussi discret que possible et pouvoir s'adapter à des patients de morphologies très différentes. Il convient en outre, qu'après apprentissage par le personnel soignant, les patients puissent procéder eux-mêmes à la mise en place des poches de recueil sur le porte-poches, ce qui implique, bien entendu, et en particulier pour des utilisateurs d'un certain âge, que les manipulations d'assemblage et de séparation de la poche et du porte-poches soient aussi simples et aisées que possible.

Aussi, pour tenter d'apporter une solution au problème posé, a-t-on récemment proposé, par exemple dans FR-A1-2 626 464, un système deux-pièces dans lequel les moyens d'accouplement de la poche et du porte-poches sont constitués par un collet déformable et une bague fendue agencée pour serrer le pourtour de ce collet, une tubulure ayant une dimension extérieure sensiblement égale à la dimension intérieure du collet pouvant être insérée ou retirée en coulissant dans le collet quand la bague est dans une position d'ouverture, de sorte que, quand la bague est en position serrée autour du collet, celui-ci est déformé autour de la tubulure. La réalisation de l'ensemble est relativement complexe, étant donné que les diamètres de la tubulure et du collet doivent être réalisés avec de très faibles tolérances dimensionnelles pour que l'assemblage à étanchéité soit obtenu par une déformation réduite du collet dont la section en U fait normalement obstacle à cette déformation. Des difficultés analogues existent avec les très nombreux systèmes proposés du type à emboîtement, comme décrit par exemple dans EP-A-0 171 255, ou du type de celui décrit ci-avant, et on comprend alors que, nonobstant la diversité des solutions déjà connues, le problème existe encore de fournir un appareillage pour stomie perfectionné qui permette de remplir les différentes exigences requises pour ces appareillages, d'une part, et qui permette également, d'autre part, d'orienter la poche par rapport au porte-poches, comme cela peut être souhaité pour accroître le confort de l'utilisateur. Bien que ce dernier problème ait déjà été considéré, par exemple dans EP-163 979, les solutions apportées ne sont pas entièrement satisfaisantes, soit en ce qui concerne le mode d'assemblage, soit en ce qui concerne l'étanchéité et/ou le dimensionnement des dispositifs de liaison de la poche à son support.

C'est, par conséquent, un but général de l'invention de fournir un tel appareillage applicable aux stomies digestives ou urinaires qui satisfasse ces conditions, dans lequel la mise en place de la poche sur le porte-poches préalablement fixé au corps d'un utilisateur soit extrêmement aisée, d'une part, et s'effectue, d'autre part, pratiquement sans application de pression dans la zone voisine de la stomie.

C'est, aussi, un but de l'invention de fournir un tel appareillage qui soit d'une grande sécurité d'utilisation et dans lequel, en particulier, l'étanchéité de liaison de la poche au porte-poches soit renforcée lors d'une augmentation de pression dans la poche.

Le problème est résolu dans un appareillage pour stomie du type deux-pièces comportant un porte-poches destiné à être fixé au corps de l'utilisateur à l'aide d'un patin muni d'un adhésif ou d'une gomme adhésive sensible à la pression, ou par un moyen équivalent, ainsi qu'une poche de recueil des fluides et/ou déchets corporels propre à être assemblée de manière amovible au porte-poches par un nez qu'elle présente par le fait que, selon l'invention, la fixation de la poche sur le porte-poches résulte de la déformation d'un moyen d'étanchéité dont on accroît la dimension radiale par manoeuvre d'un dispositif d'actionnement approprié.

Dans un tel appareillage, par conséquent, et contrairement au dispositif connu selon FR-A1 2 626 464 ou aux dispositifs du type à emboîtement, aucune pression n'est exercée dans le voisinage de la stomie lors de la mise en place de la poche sur le porte-poches étant donné que l'ouverture du nez de poche est de plus grande dimension que celle de la partie correspondante du porte-poches avec laquelle elle coopère et qui entoure la stomie dans sa condition d'utilisation.

Dans un appareillage selon l'invention, complémentairement, le nez de poche est intérieur à la poche proprement dite de sorte qu'il évite, par association avec le porte-poches, tout effet de "collage" des feuilles constitutives de la poche, "collage" qui s'oppose parfois à l'évacuation satisfaisante des déchets corporels.

Dans une première forme de réalisation d'un appareillage selon l'invention, le dispositif d'actionnement est un anneau fendu et audit anneau est associé le moyen d'étanchéité constitué par un joint d'étanchéité en matériau élastique interposé entre le nez de poche et l'anneau lorsque la poche est rapportée sur le porte-poches.

Dans un mode d'exécution de cette première forme de réalisation, l'anneau fendu est d'une pièce avec un moyen d'actionnement avantageusement constitué par un levier articulé sur l'anneau et qui commande un mécanisme du type "à genouillère".

L'anneau fendu et le moyen d'actionnement sont de préférence moulés en une matière plastique douée de propriétés mécaniques et élastiques telles qu'elles permettent de réaliser les articulations du levier et du mécanisme "à genouillère" par effet de charnière.

Dans ce mode d'exécution, également, la pièce unique constitutive de l'anneau fendu et de son moyen d'actionnement est moulée dans sa condition correspondante à la grande dimension de l'anneau fendu.

Dans une autre réalisation, le dispositif d'actionnement est constitué par deux demi-anneaux auxquels est associé le joint d'étanchéité en matériau élastique interposé entre le nez de poche et les demi-anneaux lorsque la poche est rapportée sur le porte-poches.

Chaque demi-anneau est de préférence d'une pièce avec le moyen d'actionnement constitué par un levier articulé sur lui et qui commande un mécanisme à genouillère.

Dans une autre réalisation, les deux demi-anneaux sont réunis entre eux par un mécanisme "à genouillère" lui même constitué par deux bras articulés entre eux autour d'une charnière et sur les demi-anneaux, respectivement, par des charnières dont la première forme aussi l'articulation du levier.

En variante, les deux demi-anneaux sont réunis entre eux par deux mécanismes à genouillères constitués chacun par deux bras articulés entre eux autour d'une charnière et sur les demi-anneaux par des charnières, les articulations des leviers étant réalisées autour de deux axes charnières solidaires de la collerette par une pièce.

Selon une autre caractéristique de l'invention, le joint d'étanchéité est à section droite de forme trapézoïdale et enserre un retour de l'anneau fendu, la déformation de ce dernier lors de la manoeuvre du moyen d'actionnement entraînant celle du joint jusqu'à application de celui-ci à étanchéité sur le nez de poche.

Ce dernier est avantageusement en une matière plastique suffisamment rigide pour encaisser l'effort de serrage développé par le joint d'étanchéité avec lequel il coopère par sa face interne à section de forme générale hyperbolique dans la condition d'assemblage de la poche et du porte-poches.

Pour le montage du dispositif d'actionnement sur le porte-poches ce dernier présente une gorge limitée par un collet, -qui entoure la stomie dans la condition d'utilisation de l'appareillage-, et par deux collerettes sensiblement parallèles et perpendiculaires à l'axe du collet, une première collerette coopérant au maintien du joint d'étanchéité et la seconde servant à la solidarisation du porte-poches et du patin, d'une part, ainsi qu'au guidage et/ou à la retenue du dispositif d'actionnement sur ledit porte-poches, d'autre part.

Pour assurer cette dernière fonction on prévoit, le plus simplement, que la seconde collerette soit munie de lumières dans lesquelles sont logées des tétons de l'anneau fendu.

Dans une autre forme de réalisation, le moyen d'étanchéité est un anneau fendu mais conformé pour ménager sur sa périphérie des saillies propres à coopérer avec une rainure ou des évidements de forme conjuguée prévue(s) sur le nez de poche.

Dans une telle réalisation l'anneau fendu est monté sur le porte-poches dans une gorge que présente un embout de ce dernier, l'embout comportant une collerette à face externe tronconique avec laquelle est propre à coopérer la face de même conicité du nez de poche et le moyen d'actionnement est avantageusement constitué par un levier qui déplace une des extrémités de l'anneau fendu lequel comporte des plots ou analogues dans lequel viennent s'engager des creux du levier permettant de maintenir ce dernier, avec verrouillage, dans la condition d'assemblage de la poche sur le porte-poches.

Dans encore une autre forme de réalisation, c'est un même organe élastiquement déformable qui joue le rôle de joint d'étanchéité, par lui même ou par une bague qui lui est directement associée, et de dispositif d'actionnement.

Dans un mode d'exécution de cette forme de réalisation, l'organe mis en oeuvre est un anneau déformable à l'aide d'un fluide, pneumatique ou hydraulique, pour accroître sa dimension radiale lors de la fixation à étanchéité du nez de poche sur le porte-poches.

Dans une autre exécution, l'anneau est une chambre torique gonflable et dégonflable par l'intermédiaire d'une valve qui lui est associée, ladite chambre formant elle-même joint d'étanchéité ou comportant, en variante, un joint d'étanchéité auxiliaire.

L'invention prévoit, en tant que moyen de gonflage, un dispositif du type seringue, ou une réserve de fluide gazeux sous pression, comme une bombe à air comprimé portative ou encore un dispositif hydraulique dans lequel le déplacement d'un levier permet de transférer un volume donné de liquide dans et hors de la chambre à gonfler.

L'appareillage pour stomie selon l'invention est ainsi caractérisé en ce qu'il est constitué, quoi-qu'appartenant au type des systèmes "deux-pièces", par au moins trois éléments à savoir, un porte-poches comportant un manchon ou collet, une poche à nez de poche relativememt rigide et un organe élastiquement déformable agissant pour immobiliser le nez de poche par rapport au collet lorsque les dimensions dudit organe sont accrues par un moyen d'actionnement mécanique, hydraulique ou pneumatique.

Dans une autre forme de réalisation d'un appareillage selon l'invention, on accroît la dimension radiale d'un moyen d'étanchéité comprenant un boudin d'étanchéité proprement dit, monté à déplacement radial par rapport au manchon du porte-poche et vers l'extérieur dudit manchon lorsqu'on augmente le volume d'une chambre adjacente audit boudin.

Dans un premier mode d'exécution de cette forme de la réalisation, l'augmentation de volume de la chambre adjacente au boudin d'étanchéité résulte de l'introduction dans ladite chambre d'une quantité de fluide supplémentaire par rapport à celle qu'elle contenait initialement.

Cette introduction peut être obtenue par des moyens mécaniques, comme une déformation locale à l'aide d'un levier ou analogue d'une capacité de volume donné ou, en variante, par apport dans ladite chambre, directement ou indirectement, d'un fluide hydraulique ou pneumatique à partir d'une source de fluide sous pression, comme une seringue, une pompe, un réservoir de gaz comprimé, etc...

Selon une autre caractéristique de l'invention, ladite capacité est limitée par une paroi en matériau élastique, sur laquelle est propre à agir un piston dont le mouvement est commandé par l'actionnement d'un ou de levier(s).

Selon encore une autre caractéristique de l'invention, la capacité est enfermée dans un boîtier solidaire du porte-poches par des potences et ces dernières présentent des crochets avec lesquels sont propres à coopérer des crochets conjugués du ou des levier(s) d'actionnement du piston pour le maintien de ce ou ces levier(s) dans leur position correspondant à celle d'assemblage de la poche au porte-poches.

Dans un autre mode de réalisation, la capacité permettant d'alimenter la chambre de volume variable adjacente au boudin d'étanchéité peut être reliée à une source de fluide sous pression par un dispositif de valve avec interposition d'un tube souple déformable.

Dans un mode d'exécution, le dispositif de valve est relié à un clapet, lui même abouché à un tube du type soufflet pour gonfler la capacité par un moyen analogue à une pompe.

Dans une autre réalisation de l'invention, le moyen d'étanchéité est un élément plein ou creux comportant des lèvres ou membranes de fixation sur un corps solidaire du porte-poches, lequel ménage des moyens permettant de modifier le volume de la chambre adjacente audit boudin d'étanchéité.

Dans un mode d'exécution de cette réalisation, le corps présente des canaux débouchant dans une enceinte dont le volume peut être modifié et/ou dans laquelle peut être introduit un fluide sous pression.

Dans encore une autre réalisation, on accroît la dimension radiale du moyen d'étanchéité à l'aide d'un anneau fendu agissant par un effet de "coin" par l'intermédiaire d'une rondelle adjacente audit moyen d'étanchéité, lequel est alors avantageusement constitué par un profilé torique à section droite quelque peu trapézoïdale, enfermant une cavité annulaire remplie de fluide liquide ou gazeux.

En variante, le moyen d'étanchéité est un profilé plein en un matériau cellulaire à peau de surface intégrale, comme obtenu par les techniques connues de mise en oeuvre des matériaux du type mousse ou cellulaire de l'industrie des élastomères et/ou des plastomères.

Dans une autre variante, le moyen d'étanchéité est un anneau plein ou creux à section droite polygonale en un matériau du type élastomère de faible dureté, avantageusement de 20 à 40 Shore A.

Lorsque l'appareillage comporte un anneau fendu pour accroître la dimension radiale du moyen d'étanchéité, l'anneau présente avantageusement à ses extrémités des pattes d'actionnement permettant le rapprochement desdites extrémités ainsi que des moyens de becs, crochets, ergots ou analogues, pour le maintien en position "ouverte" ou "fermée" dudit anneau fendu.

L'invention prévoit encore, complémentairement, d'associer audit anneau fendu une languette solidaire d'une des pattes, logée à coulissement dans un passage du; l'autre patte pour le guidage l'une par rapport à l'autre desdites pattes lors de leur mouvement de rapprochement et/ou d'éloignement.

Quel que soit le mode de réalisation, l'invention prévoit que le moyen d'étanchéité soit associé à un support qui peut être monté à rotation par rapport au manchon du porte-poches, de sorte qu'après que la poche ait été rapportée sur ce dernier elle peut être orientée par rapport au porte-poches, comme cela est parfois souhaité pour accroître le confort de l'utilisateur.

En variante, le support du moyen d'étanchéité n'est pas monté à rotation par rapport au manchon du porte-poches et l'orientation de la poche par rapport au porte-poches peut être effectuée, -alors que la poche est montée sur le porte-poches-, après une légère diminution de la dimension radiale du moyen d'étanchéité qui abaisse la pression d'application de la poche sur le porte-poches.

Dans une réalisation avantageuse, ce dernier présente un flasque qui maintient le moyen d'étanchéité à l'encontre d'un déplacement axial, le diamètre interne du nez de poche étant plus petit que le diamètre externe dudit flasque, mais plus grand que le diamètre externe du moyen d'étanchéité.

L'invention a aussi pour objet un porte-poches propre à entrer dans la constitution d'un tel dispositif.

L'invention sera bien comprise par la description qui suit, faite à titre d'exemple et en référence au dessin annexé, dans lequel :
- la figure 1 est une vue schématique en perspective montrant, distant l'un de l'autre, un porte-poches et une poche d'un appareillage selon l'invention ;
- la figure 2 est une vue partielle en coupe longitudinale d'un porte-poches et d'une poche d'un appareillage selon l'invention pour une première forme de réalisation ;
- la figue 3 est une vue analogue à celle de la figure 2 montrant la poche rapportée sur le porte-poches mais avant fixation ;
- la figure 4 est une vue analogue à celle de la figure 3 mais après fixation de la poche sur le porte-poches ;
- la figure 5 est une vue partielle analogue à celle de la figure 3 mais pour une autre forme de réalisation ;
- la figure 6 est une vue analogue à celle de la figure 4 pour la réalisation montrée sur la figure 5 ;
- la figure 7 est une vue analogue à celle des figures 4 et 6 mais pour encore une autre forme de réalisation ;
- la figure 8 est une vue en élévation d'une partie constitutive d'un appareillage selon l'invention pour encore une autre forme de réalisation et dans une première condition ;
- la figure 9 est une vue analogue à celle de la figue 8 mais pour une autre condition ;
- la figure 10 est une vue partielle, en perspective, de cette partie ;
- la figure 11 est une vue en élévation d'une partie constitutive d'un appareillage selon l'invention pour encore une autre forme de réalisation et dans une première condition ;
- la figure 12 est une vue analogue à celle de la figure 11 mais pour une autre condition ;
- la figure 13 est une vue partielle analogue à celle de la figure 11, mais à plus grande échelle et pour une variante ;
- la figure 14 est une vue partielle analogue à celle de la figure 12 mais pour la variante montrée sur la figure 13 ;
- la figure 15 est une vue schématique en coupe selon la ligne 15-15 de la figure 14 ;
- la figure 16 est une vue schématique, en élévation, d'un porte-poches d'un appareillage selon l'invention pour une autre forme de réalisation ;
- la figure 17 est une vue en coupe selon la ligne 17-17 de la figure 16, mais à plus grande échelle ;
- la figure 17A est une vue analogue à celle de la figure 17, mais pour une variante de réalisation ;
- la figure 17B illustre la mise en place d'une poche sur un porte-poche ;
- la figure 18 est une vue en coupe selon la ligne 18-18 de la figure 16 ;
- la figure 19 est une vue partielle analogue à celle de la figure 16 mais pour une autre forme de réalisation ;
- la figure 20 est une vue en coupe selon la ligne 20-20) de la figure 19 ;
- la figure 21 est une vue analogue à celle des figures 16 et 19 mais pour encore une autre forme de réalisation ;
- la figure 22 est une vue en coupe selon la ligne 22-22 de la figure 21 ;
- la figure 23 est une vue analogue à celle des figures 19 et 21, mais pour une autre forme de réalisation ;
- la figure 24 est une vue en coupe selon la ligne 24-24 de la figure 23 ;
- la figure 25 est une vue en coupe selon la ligne 25-25 de la figure 23, mais à plus grande échelle ;
- la figure 26 est une section selon la ligne 26-26 de la figure 23 ;
- la figure 27 est une vue schématique analogue à celle de la figure 25, mais pour une variante de réalisation ;
- la figure 28 est une vue analogue à celle des figures 19 et 21, mais pour encore une autre forme de réalisation ;
- la figure 29 est une vue schématique partielle en coupe selon la ligne 29-29 de la figure 28, mais à plus grande échelle ;
- la figure 30 est une vue analogue à celle de la figure 28, mais pour une variante de réalisation.

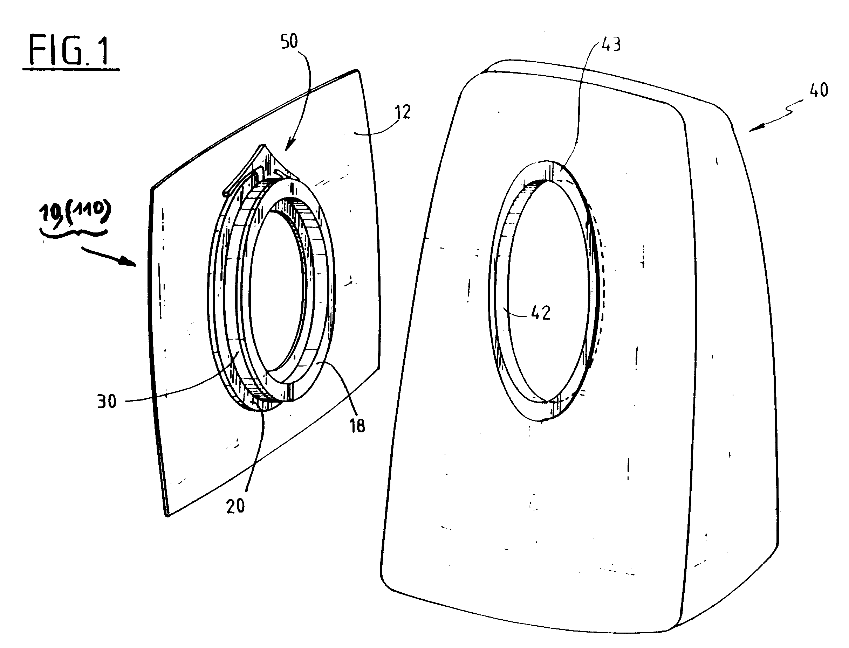

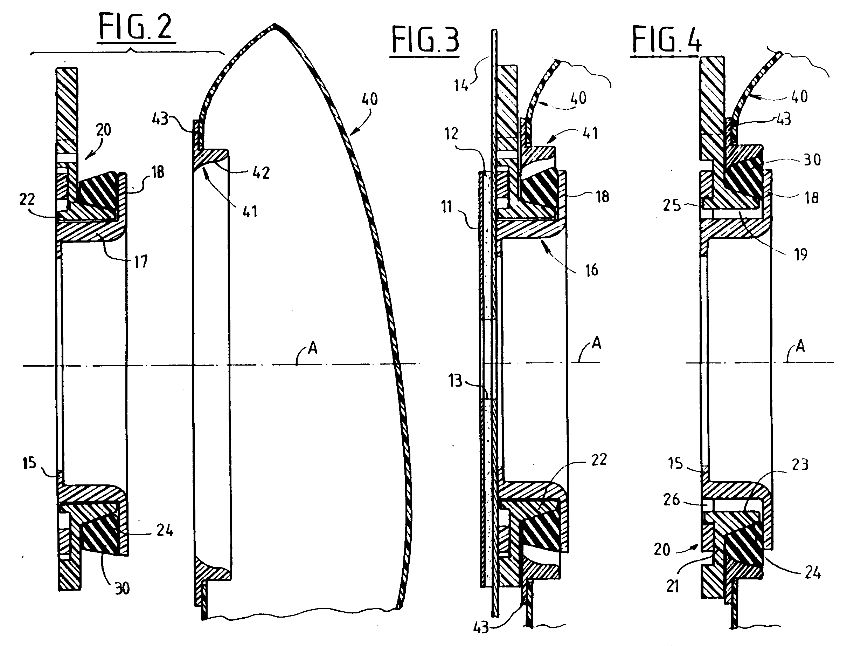

On se réfère d'abord aux figures 1 à 4 qui montrent une première forme de réalisation d'un appareillage pour stomie selon l'invention du type "deux-pièces", c'est-à-dire comprenant un porte-poches 10 prévu pour être fixé autour d'une ouverture artificielle du corps d'un utilisateur à l'aide d'un moyen de fixation approprié, comme un patin muni d'un adhésif ou d'une gomme adhésive sensible à la pression 12, en soi connu, et qui est protégé aussi longtemps que le dispositif n'est pas mis en oeuvre par une pellicule 11, figure 3, qui peut être retirée par pelage ; en variante et/ou complémentairement, le porte-poches 10 peut être maintenu sur le corps de l'utilisateur à l'aide d'une ceinture non représentée ou d'un moyen analogue. Le porte-poches 10, dont le patin 12 présente une ouverture 13 entourant la stomie dans sa condition d'utilisation comprend également, sur la face du patin 12 opposée à celle venant au contact du corps de l'utilisateur, une feuille composite adhésive 14 qui contribue à la protection de la zone péristomiale et au maintien sur la peau de l'utilisateur. Sur la face de la feuille composite 14 opposée à celle de liaison au patin 12 est fixée par une collerette 15 un embout 16 comprenant un manchon ou collet cylindrique 17 d'axe A et une autre collerette 18 sensiblement parallèle à la colleette 15, -et ainsi perpendiculaire comme cette dernière à l'axe A-, les deux collerettes 15, 18, ménageant entre elles une gorge 19.

Cette dernière est prévue pour le montage d'un anneau déformable, 20, par exemple un anneau fendu comprenant un corps sensiblement plan 21 à contour circulaire limité intérieurement par un retour 22 à surface interne 23 cylindrique et à surface externe 24 tronconique, figures 2 et 4. L'anneau 20 qui, selon l'invention, est le dispositif assurant la fixation d'une poche 40 sur le porte-poches 10 par un accroissement de ses dimensions (d'une manière qui sera explicitée ci-après) est retenu et guidé par rapport à la collerette 15 par des tétons 25 qu'il présente et qui sont logés dans des lumières 26 de ladite collerette 15.

Sur le retour 22 de l'anneau déformable 20 est rapportée une bague ou joint d'étanchéité 30 en matériau élastique, avantageusement un élastomère thermoplastique (TEP), à section droite de forme générale trapézoïdale et dont l'épaisseur, mesurée parallèlement à l'axe A, est sensiblement égale à la distance séparant les collerettes 15 et 18 diminuée de l'épaisseur du corps 21 de l'anneau de sorte que la bague 30 trouve, elle aussi, place dans la gorge 19. Le plus grand diamètre nominal externe de la bague 30 est inférieur au plus petit diamètre du nez de poche 41 de la poche 40 de sorte que la poche peu être mise en place avec son nez de poche entourant la bague ou joint d'étanchéité 30 et cela sans appliquer de pression dans la zone voisine de la stomie. Pour assurer la fixation de la poche 40 sur le porte-poches 10 il suffit alors de déformer l'anneau 20 pour accroître ses dimensions et, partant, celles de la bague 30 jusqu'à son appui à force avec un effet de serrage contre la surface interne 42 (à section droite de forme générale hyperbolique) du nez de poche 41 dirigé vers l'intérieur de la poche 40 et réalisé en un matériau relativement rigide, par exemple du polyéthylène haute densité ou du polypropylène.

Pour ce faire et amener l'appareillage dans la condition montrée sur la figure 4, l'invention prévoit d'associer à l'anneau déformable 20 un moyen d'actionnement approprié. Dans la forme de réalisation décrite et représentée le moyen d'actionnement 50 est réalisé en tant que levier permettant d'écarter les extrémités adjacentes de l'anneau fendu et de verrouiller le dispositif en cette position opératoire, le plus simplement en faisant coopérer des plots ou analogues de la périphérie de l'anneau avec des creux du bord interne du levier.

Une manoeuvre du levier 50 en sens opposé, c'est-à-dire ramenant l'anneau fendu 20 en sa condition initiale ce petite dimension permet de retirer la poche 40 du porte-poches 10.

La poche 40 pour le recueil des fluides et/ou déchets corporels propres à être évacués au travers de l'embout 16 peut être du type à jeter ou à vider et est réalisée en solidarisant par collage, soudure ou analogue, une collerette externe 43 du nez de poche 41 à la poche proprement dite constituée en un film de polyéthylène ou de PVC, ou de polyamide, ou en un film barrière complexe de type polyéthylène/EVA/polychlorure de vinyle/EVA/polyéthylène, comme ceux connus sous la marque déposée SARANEX de la Société DOW CHEMICAL ou un film complexe du type EVA/copolymère polychlorure de vinylidène-EVA, comme ceux connus sous la marque CRYOVAC de la Société GRACE ou encore un film complexe du type EVA/EVOH/EVA, ou un matériau analogue.

Le fonctionnement de l'appareillage est simple et sûr et un fonctionnement très satisfaisant a été obtenu pour une réalisation comprenant un embout 16 d'environ 55 mm de diamètre, un nez de poche d'environ 68 mm de diamètre interne et une bague ou joint d'étanchéité d'environ 66 mm de diamètre externe.

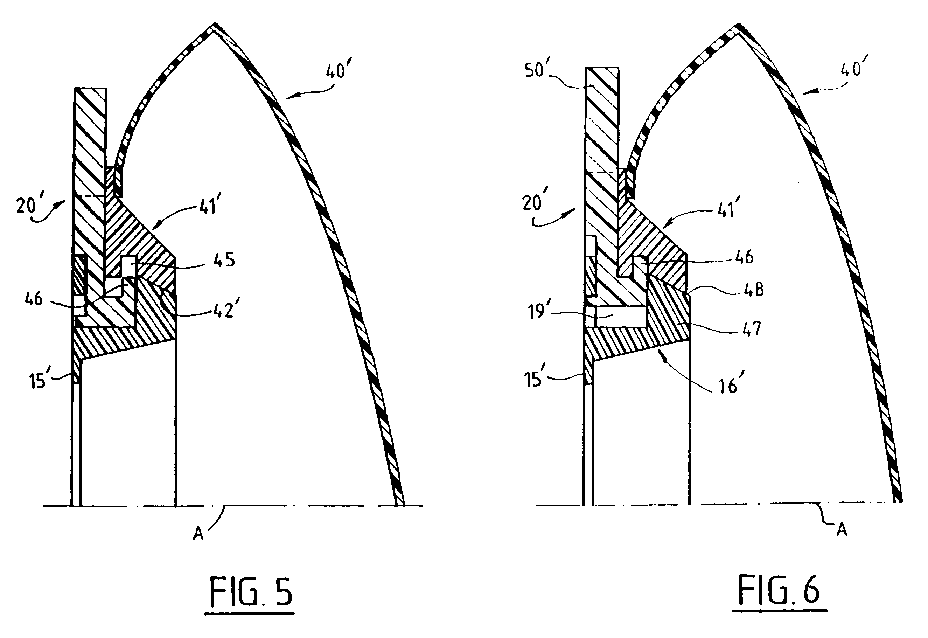

On se réfère maintenant aux figures 5 et 6 qui illustrent schématiquement une autre forme de réalisation. Dans celle-ci, la poche 40′ est fixée par soudure ou collace sur un nez de poche 41′ dirigé vers l'intérieur de la poche et conformé suivant une surface intérieure 42′ tronconique dont la grande base est découpée suivant des évidements ou une rainure 45. Avec ces évidements ou rainure 45 sont propres à coopérer des saillies 46, radiales, d'un organe déformable 20′ monté sur le porte-poches dans une gorge 19′ que présente un embout 16′. Ce dernier, qui comprend une collerette 15′ analogue à la collerette 15 de l'embout de la forme de réalisation décrite ci-dessus, est prévu pour coopérer avec le nez de poche 41′ par une nervure 47 à surface extérieure 48 tronconique et de même conicité que celle de la surface 42′ dudit nez de poche. Dans cette réalisation, l'organe 20′ déformable par élargissement est également un anneau fendu en matière plastique, avantageusement moulé d'une pièce avec un moyen d'actionnement 50′ permettant d'accroître ses dimensions pour passer de la condition montrée sur la figure 5 à la condition montrée sur la figure 6 qui est celle de fixation de la poche 40′ sur le porte-poches.

Pour la mise en oeuvre d'un tel appareillage, le porte-poches est d'abord fixé au corps de l'utilisateur à l'aide du patin 12 muni d'un adhésif ou d'une gomme adhésive et de la feuille 14 après qu'ai été retirée, bien entendu, la pellicule de protection 11. Pour la mise en place de la poche 40′ celle-ci est présentée en regard du porte-poches et le nez de poche 41′ est emboîté sur la nervure 47, pratiquement sans application de pression dans la zone voisine de la stomie. Après actionnement du levier 50′ qui, en augmentant les dimensions de l'anneau 20′, fait pénétrer les saillies 46 de celui-ci dans les évidements ou la rainure 45, la poche 40′ est maintenue à étanchéité sur le porte-poches par coopération des surfaces coniques 42′ et 48.

Pour séparer la poche 40′ du porte-poches, c'est un processus inverse qui est mis en oeuvre après que le levier 50′ ait été d'abord déverrouillé puis manoeuvré en direction opposée de celle décrite ci-dessus.

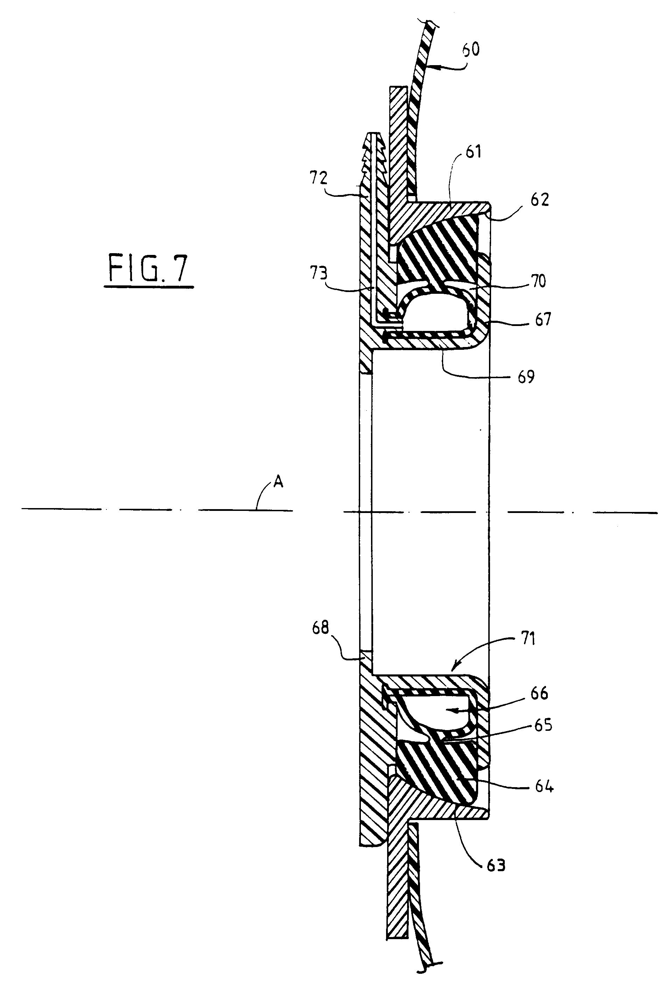

L'invention ne se limite nullement aux modes de réalisation et d'application qui viennent d'être décrits. Ainsi, et comme montré sur la figure 7, elle prévoit ces formes de réalisation dans lesquelles la fixation de la poche sur le porte-poches résulte de la déformation d'un organe dont on accroît les dimensions à l'aide d'un fluide pneumatique ou hydraulique. Dans le mode d'exécution représenté, la poche 60 comporte un nez de poche 61 dirigé vers l'intérieur de la poche et dont la surface interne 62 est incurvée, par exemple suivant un contour hyperbolique. Avec cette surface est propre à coopérer la surface externe 63 d'une bague d'étanchéité 64, à section droite trapézoïdale, en matériau élastique, par exemple en élastomère thermoplastique (TEP), reliée par un pontet 65 à une chambre torique 66 logée dans la gorge 70 formée sur un embout 71 solidaire du porte-poches entre un collet cylindrique d'axe A et deux collerettes externes et internes 67 et 68, respectivement, sensiblement parallèles entre elles et perpendiculaires à l'axe A. La chambre 66 élastiquement déformable et avantageusement en le même matériau que celui de la bague 64 est reliée à un embout 72 percé d'un canal traversant 73 sur lequel est interposée une valve, non représentée.

Le fonctionnement de cette forme de réalisation résulte immédiatement de ce qui précède. Le porte-poches étant fixé au corps de l'utilisateur et la chambre 66 dégonflée, la poche 60 est présentée en regard du porte-poches et le nez de poche 61 est mis en place sans difficulté sur la bague 64. A l'embout 72 solidaire du porte-poches est alors abouché un moyen 66′ de gonflage, hydraulique ou pneumatique, comme un dispositif de seringue, une réserve de fluide gazeux sous pression telle qu'urne bombe à air comprimé portative ou un dispositif dans lequel le déplacement d'un levier permet de transférer au travers du canal 73 un volume donné de liquide dans la chambre 66. Cette dernière augmente alors de volume et applique, sous pression, la bague 64 contre le nez de poche 61 pour la fixation étanche de la poche 60 sur le porte-poches.

C'est un processus inverse qui est mis en oeuvre pour séparer la poche du porte-poches après que la pression du fluide hydraulique ou pneumatique dans la chambre 66 ait été supprimée.

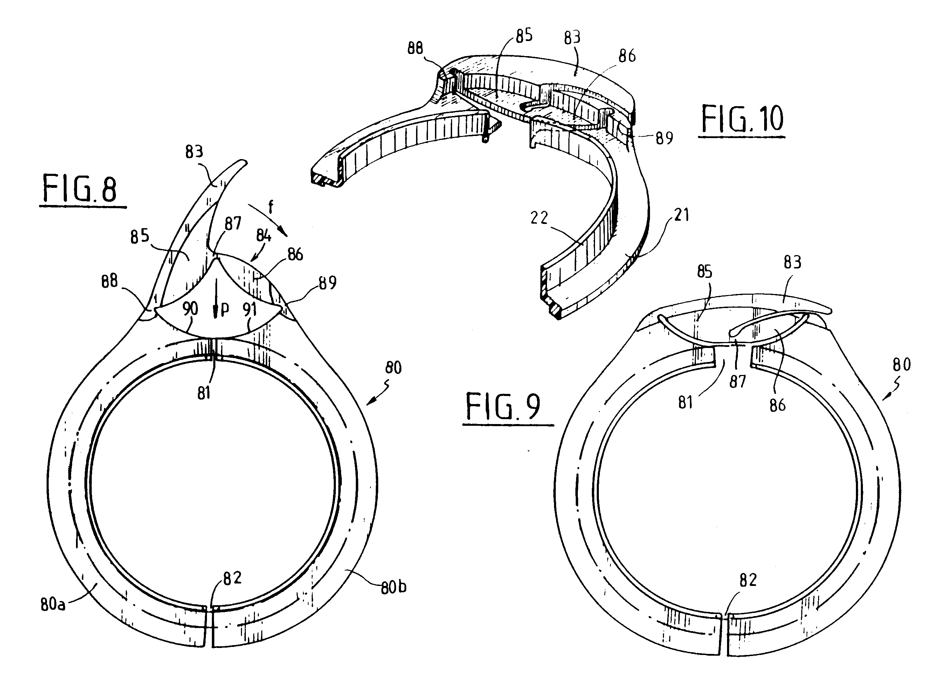

On se réfère maintenant aux figures 8 à 10 illustrant une forme de réalisation préférée d'un appareillage selon l'invention dans lequel le dispositif assurant la fixation de la poche sur le porte-poches est un anneau fendu en matière plastique moulé d'une pièce avec le moyen d'actionnement de l'anneau. Dans cette forme de réalisation, la structure de la poche et celle du porte-poches sont sensiblement celles de la réalisation décrite en référence aux figures 1 à 4 et les mêmes références y désignent des parties semblables. Comme bien visible sur les figures 8 et 9, l'anneau 80 est fendu en deux zones diamétralement opposées 81 et 82 et les deux demi-anneaux 80ₐ et 80_{b} sont réunis entre eux par un levier 83 et un mécanisme du "type à genouillère", 84, dont les deux branches 85 et 86 sont articulées entre elles autour d'une charnière 87, d'une part, et sont également articulées, d'autre part, autour d'une charnière 88 sur le demi-anneau 80ₐ et autour d'une charnière 89 sur le demi-anneau 80_{b}, la charnière 88 formant également l'axe de pivotement du levier 83. Celui-ci est moulé d'une pièce avec l'anneau fendu 80 dans la conditi4on de plus grande dimension de ce dernier, qui est celle montrée sur la figure 9, en laquelle le levier 83 est rabattu au contact du bras 86 lequel est alors placé sensiblement co-circulairement au corps 21 de l'anneau, -comme l'est également le bras 85-, alors que dans la condition de petite dimension, montrée sur la figure 8, le levier 83 est les bras 85 et 86 font saillie par rapport audit anneau.

Pour faciliter l'actionnement du levier 83 et accroître la manoeuvrabilité du dispositif, le levier et les zones d'articulations 88 et 89 sont moulées avec des surépaisseurs par rapport au corps 21, au retour 22 et au voile formant les bras 85 et 86, lesquels sont en outre incurvés en correspondance de découpes 90 et 91 des demi-anneaux 80ₐ et 80_{b} pour offrir un logement auxdits bras, figures 9 et 10.

Le fonctionnement de cette forme de réalisation est analogue à celui de la réalisation décrite aux figures 1 à 4. Le porte-poches étant fixé au corps de l'utilisateur par le patin 12, le levier 83 est amené dans la condition montrée sur la figure 8 : dans cette condition, la poche 40 peut être présentée en regard du porte-poches et le nez de poche 41 rapporté sans effort et sans difficulté sur la bague 24. Lorsqu'on fait alors pivoter le levier 83 dans le sens de la flèche f, autour de l'articulation à charnière 88, figure 8, on déplace l'articulation 87 dans le sens de la flèche p, ce qui écarte l'une de l'autre les deux extrémités des demis-anneaux 80ₐ et 80_{b} voisines de la fente 81. A fin de pivotement du levier 83 la condition est celle montrée sur la figure 9 en laquelle la bague 24 est au contact du nez de poche 41 pour assurer la fixation étanche de la poche sur le porte-poches.

Pour séparer la poche du porte-poches, on amène par un pivotement inverse de celui qui vient d'être décrit le levier 83 de la condition montrée sur la figure 9 à celle montrée sur la figure 8 ce qui rétablit l'anneau fendu 80 dans sa condition de petite dimension et la bague en matériau élastique 24 dans sa condition initiale autorisant ainsi le retrait du nez de poche 41 par écartement du porte-poches.

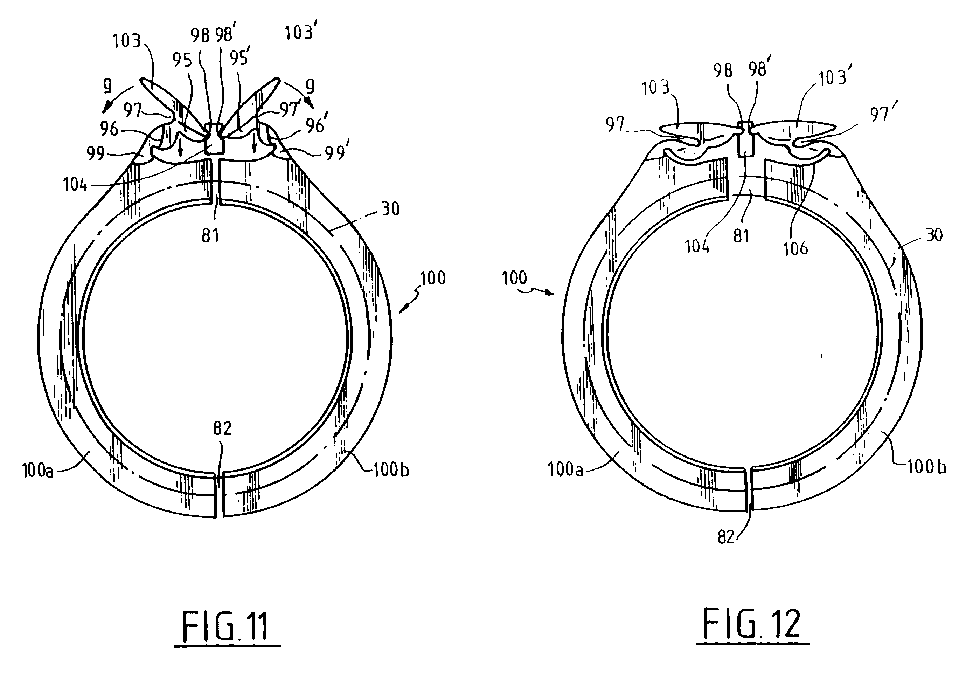

Les figures 11 et 12 illustrent une autre forme de réalisation, similaire à celle des figures 8 à 10, avec, toutefois un dédoublement des moyens de déformation assurant un fonctionnement plus souple, mais identique dans son principe à celui de la forme de réalisation des figures 8 à 10. Dans cette forme de réalisation, le dispositif d'actionnement est un anneau 100 fendu en deux zones diamétralement opposées 81 et 82 pour ménager deux demi-anneaux 100ₐ et 100_{b} réunis entre eux par deux mécanismes à genouillère eux-mêmes constitués chacun par deux bras référencés 95, 96 pour le premier et 95' et 96' pour le second, chacun des mécanismes pouvant être actionné au moyen d'un levier 103 et 103', respectivement. Comme bien visible sur les figures 11 et 12, les bras 95 et 96 sont articulés entre eux autour d'une charnière 97, tandis que les bras 95' et 96' sont articulés autour d'une charnière 97', l'articulation sur chacun des demi-anneaux 100ₐ et 100_{b} ayant lieu par l'intermédiaire de charnières 99 et 99', respectivement. Dans cette réalisation, en outre, les leviers 103 et 103' sont articulés à pivotement autour de deux axes montrés en 98 et 98' et qui sont solidaires d'une collerette analogue à la collerette 15 de la forme de réalisation précédente par l'intermédiaire d'une pièce 104. Dans cette forme de réalisation c'est un actionnement des deux leviers 103 et 103' dans le sens des flèches g et g' qui provoque l'accroissement de dimension de l'anneau 100, figure 12, et, par suite, la fixation d'une poche sur le porte-poches, un pivotement desdits leviers en sens opposé permettant, par contre, la libération de la poche par rapport au porte-poches.

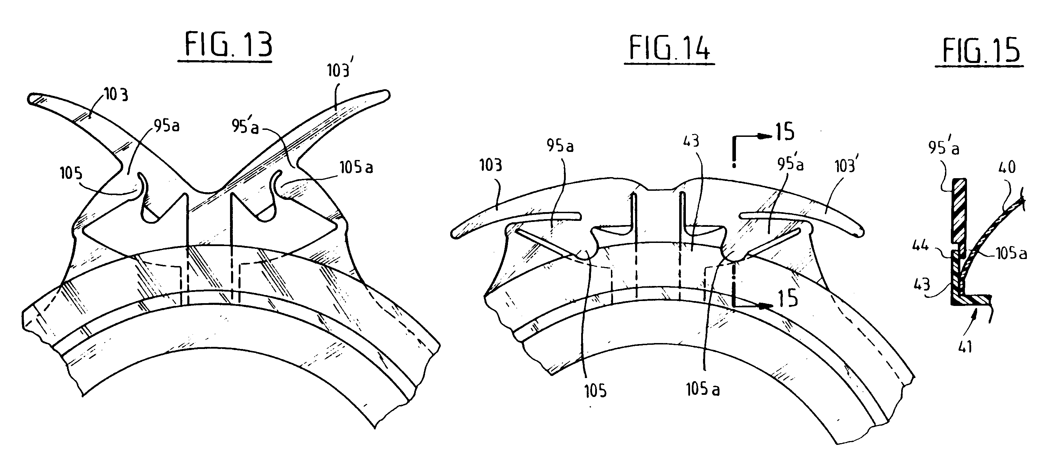

Dans la variante montrée schématiquement sur les figures 13 à 15, les bras 95a et 95'a (analogues aux bras 95 et 95' de la réalisation précédente) sont conformés pour ménager chacun une corne 105, 105a, respectivement, de plus faible épaisseur que le bras qu'elle prolonge (figure 15) et qui, de ce fait, vient chevaucher la marge périphérique 44 de la collerette 43 du nez de poche 41 (figure 14) lorsque la poche est fixée au porte-poches. La présence des cornes 105, 105a propres à coopérer avec la marge 44 de la collerette 43 contribue au maintien satisfaisant de la poche sur le porte-poches, notamment lorsque la poche se remplit et que son poids augmente.

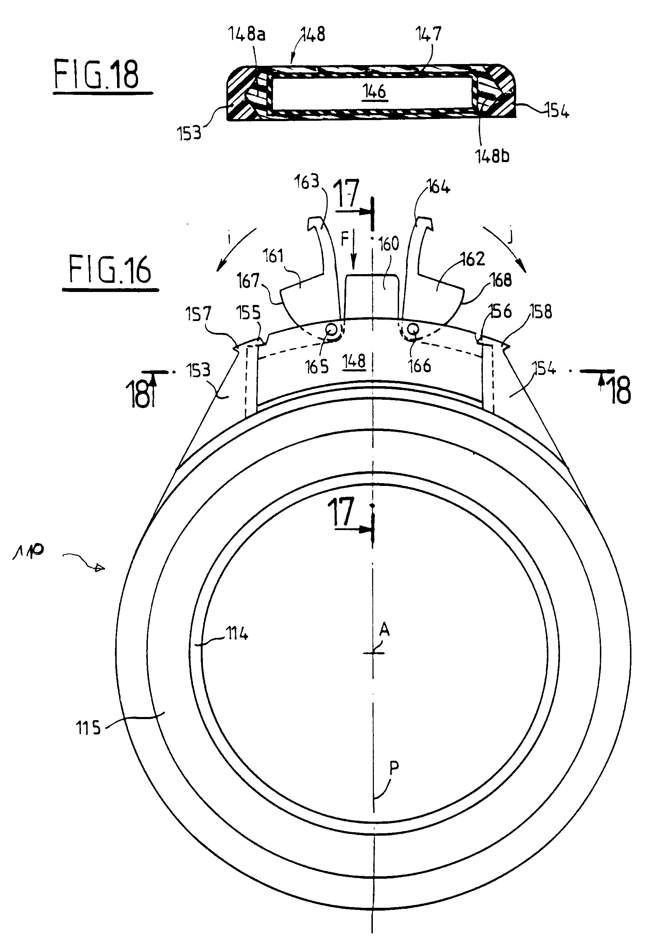

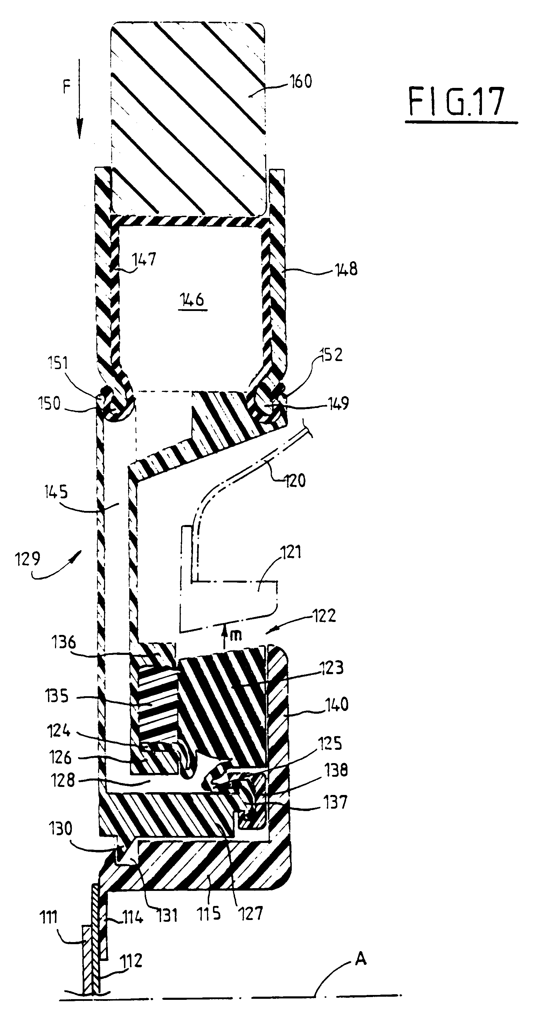

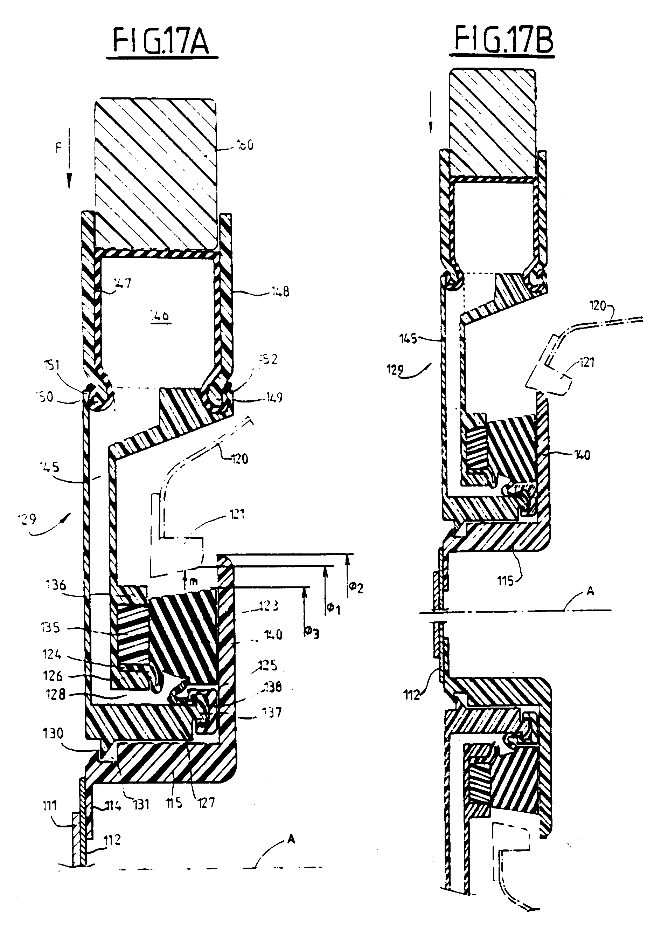

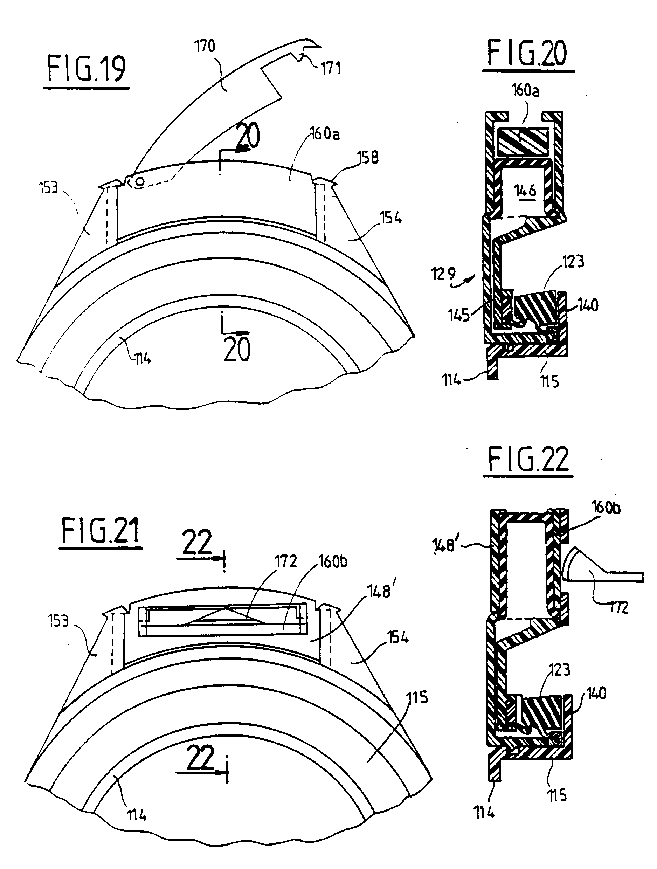

On se réfère maintenant aux figures 16 à 18 relatives à une autre forme de réalisation. Dans celle-ci la fixation de la poche 120 sur le porte-poches 110, -à patin adhésif 112 protégé par une pellicule 111-, est obtenue en accroissant la dimension radiale d'un moyen d'étanchéité 122 comprenant un boudin d'étanchéité proprement dit, 123, à section droite quelque peu trapézoïdale dont sont solidaires deux lèvres ou membranes souples, élastiques, 124 et 125, fixées sur deux parois 126 et 127 distantes l'une de l'autre du porte-poches, et délimitant entre elles une chambre 128, adjacente au boudin 123. Les parois 126 et 127 sont ménagées dans un corps 129 solidaire d'un manchon 115 du porte-poches (analogue au manchon 17 de la réalisation selon les figures 1 à 4) ou monté à rotation par rapport à ce dernier par une nervure 130 logée dans une gorge 131 dudit manchon, la membrane 124 étant fixée à la paroi 126 par une bague 135 qui serre son extrémité contre ladite paroi 126 et prend appui sur un retour 136 qui lui est parallèle, figure 17, tandis que la membrane 125 est assujettie sur le bord 137 en "champignon" de la paroi 127 par un clips 138. L'ensemble est protégé par un flasque 140, d'une pièce avec le manchon 115, et qui est ménagé à l'extrémité libre de ce dernier distante de la collerette 114 en s'étendant sensiblement parallèlement à cette dernière. Pour modifier le volume de la chambre 128, -et ainsi provoquer le déplacement du boudin 123 suivant la direction de la flèche m de façon à accroître sa dimension radiale pour l'appuyer à pression sur le nez de poche 121 quand la poche est rapportée sur le porte-poches-, l'invention prévoit de faire communiquer ladite chambre avec une capacité de volume donné, 146, par des canaux 145. Dans la réalisation décrite et représentée, la capacité 146 est ménagée par une enveloppe 147 enfermée dans un boîtier 148 fixé par ses bords périphériques 149 et 150 (qui enserrent également par un bord roulé serti les extrémités libres de l'enveloppe 147) sur des parties de formes conjuguées 151 et 152 du corps 129. Sur ce dernier sont érigées deux potences 153 et 154 symétriques par rapport à un plan diamétral p du dispositif et qui maintiennent latéralement le boîtier 148 par emboîtement de nervures à sections droites sensiblement triangulaires 148a et 148b dudit boîtier, figure 18, dans des gorges de formes conjuguées des potences, des crochets 155 et 156 desdites potences s'opposant à un mouvement radial vers l'extérieur du boîtier, lesdites potences présentant complémentairement, sur leur partie externe, des crochets 157 et 158.

Dans le boîtier 148 est monté à coulissement un piston 160, propre à être déplacé radialement en réponse à l'actionnement d'un ou de levier(s) à came(s) 161 et 162, lesdits leviers, dont les surfaces opératoires sort montrées en 167 et 168, étant munis de bras 163 et 164, respectivement, et étant montés à pivotement dans le boîtier 148 autour d'axes 165 et 166.

Pour la mise en oeuvre d'un tel appareillage, le porte-poches 110 est d'abord fixé au corps de l'utilisateur à l'aide du patin 112. Pour la mise en place d'une poche 120, celle-ci est présentée en regard du porte-poches 110 et le nez de poche 121 est placé autour du boudin d'étanchéité 123, sans application de pression dans la zone voisine de la stomie, c'est-à-dire sans douleur pour l'utilisateur. On fait alors pivoter les leviers 161 et 162 autour des axes 165 et 166, comme montré par les flèches i et j sur la figure 16 pour déplacer le piston 160 en direction de l'axe A du porte-poches, c'est-à-dire suivant la direction de la flèche F, figures 16 et 17. Ce mouvement du piston déforme l'enveloppe 147 et le fluide (gazeux ou liquide) contenu dans la capacité 146 est acheminé par les canaux 145 jusqu'à la chambre 128 dont le volume augmente en provoquant le déplacement radial, à l'écartement du manchon 115, du boudin d'étanchéité 123. Lorsque celui-ci est au contact du nez de poche 121 (réalisé en matériau relativement rigide) et qu'il prend appui sur lui, la fixation recherchée à étanchéité de la poche 120 sur le porte-poches 110 est réalisée, le maintien en condition opératoire du dispositif étant obtenu par le fait que les bras 163 et 164 des leviers 161 et 162 sont munis à leurs extrémités de crochets conjugués des crochets 157 et 158 des potences 153 et 154. Pour séparer la poche 120 du porte-poches 110, c'est un processus inverse qui est mis en oeuvre, après que les leviers 161 et 162 aient été déverrouillés puis manoeuvrés en direction opposée de celle décrite ci-dessus.

Dans la variante illustrée sur les figures 17A et 17B, le diamètre interne Φ₁ du nez de poche 121 est plus petit que le diamètre externe Φ₂ du flasque 140 mais plus grand que le diamètre externe Φ₃ du boudin 123, la hauteur du nez de poche 121 (comme mesurée parallèlement à son axe de symétrie) étant inférieure à l'épaisseur du boudin 123. Pour la mise en place de la poche, et tirant parti de la déformabilité du nez de poche 121 réalisé en matériau thermoplastique, celui-ci est inséré dans l'espace ménagé entre la face interne du flasque 140 et le retour 136, figure 17B, quelque peu à la façon dont est mis en place un pneumatique sur une jante de roue, mais sans les mêmes difficultés, bien entendu. On garantit ainsi le positionnement satisfaisant du nez de poche 121 autour du boudin d'étanchéité 123, sans risque de déplacement axial du nez de poche, d'une part, et de façon très libre autour dudit joint, d'autre part, l'opération de fixation à étanchéité pouvant ainsi être effectuée sans aucun risque d'erreur de placement du nez de poche par rapport au porte-poches.

La structure de l'appareillage illustré sur les figures 19 et 20 est très proche de celle qui vient d'être décrite et les parties correspondantes portent les mêmes références. Dans cette forme de réalisation, toute-fois, le piston 160a est soumis à l'action d'un seul levier pivotant 170 dont un crochet 171, à l'extrémité opposée à celle de pivotement, est propre à coopérer avec le crochet de forme conjuguée 158 de la potence 154.

Dans la forme de réalisation illustrée sur les figures 21 et 22 (où les parties correspondant à celles des figures 16 à 18 portent les mêmes références que sur ces figures), le piston 160b n'est pas monté à déplacement radial, mais suivant une direction parallèle à l'axe du manchon 115 lorsqu'est actionné un levier 172 qui fait pénétrer plus ou moins profondément ledit piston 160b à l'intérieur du boîtier 148′.

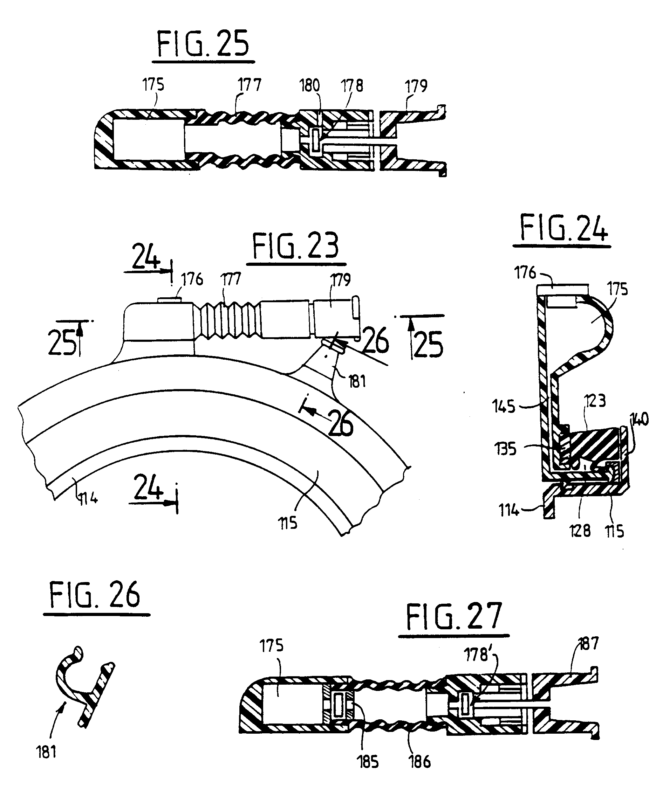

Dans la forme de réalisation illustrée sur les figures 23 à 26 où, à nouveau, les parties identiques à celles de la réalisation selon les figures 16 à 18 portent les mêmes références que dans cette dernière, les canaux 145 relient la chambre 128 à une enceinte ou capacité 175 fermée par un bouchon 176. A l'enceinte 175 est abouchée une tubulure souple, déformable, 177, sur laquelle est placée une valve 178, elle-même reliée à un embout 179) du type conique ou du type Luer pour le raccord à une source de fluide sous pression. La valve 178 est munie d'un trou évent 180 de mise à l'air libre lorsque l'embout 179 est dans une première position tandis que, dans une seconde position dudit embout, la valve 178 autorise l'introduction dans l'enceinte 175 du fluide destiné à commander le déplacement radial du boudin d'étanchéité 123 contre le nez de poche 121, lequel, comme dans les réalisations précédentes, est suffisamment rigide pour encaisser la pression d'étanchéité ainsi appliquée. Pour faciliter l'utilisation de l'appareillage, l'invention prévoit que le tube souple 177, comme un tube accordéon, puisse être maintenu à proximité du porte-poches sur lequel il est positionné par une bride 181 (lorsque la poche est fixée au porte-poches) tandis que le tube 177 peut en être écarté pour faciliter le raccord de l'embout 179 à la source de fluide sous pression.

Dans la réalisation montrée très schématiquement sur la figure 27, pour le reste identique à celle décrite immédiatement ci-dessus en référence aux figures 23 à 26, entre la valve 178' et l'enceinte 175 dans laquelle débouchent les canaux 145 sont interposés un clapet 185 de retenue de pression et un tube à soufflet, 186, l'ensemble du clapet 185 et du tube 186 constituant un système de pompe de gonflage de la chambre 128 au travers de l'enceinte 175 et des canaux 145. Dans cette réalisation, on augmente la dimension radiale du boudin d'étanchéité 123 en rapportant, par exemple par vissage, un bouton poussoir 187 sur la valve 178' puis en déformant longitudinalement le tube-soufflet 186, comme dans une pompe, la pression de gonflage, -et par conséquent celle d'application à étanchéité de la poche 120 sur le porte-poches 110-, étant maintenue après que le bouton poussoir 187 ait été retiré de la valve 178', tandis que le désassemblage de la poche et du porte-poches est obtenu en mettant l'enceinte 175 à l'air libre, le plus simplement en retirant, par dévissage, le bouchon 176.

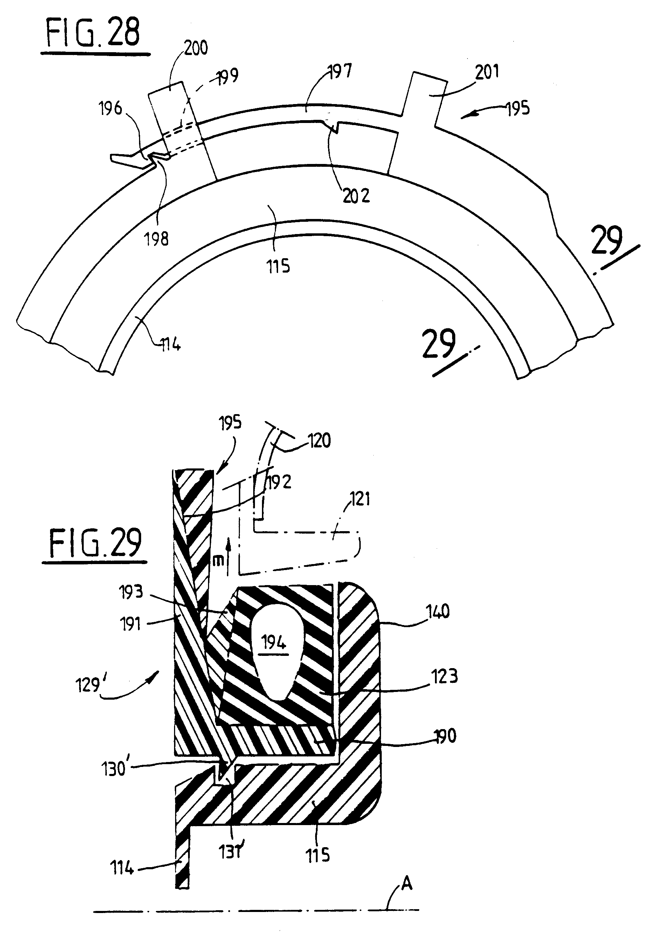

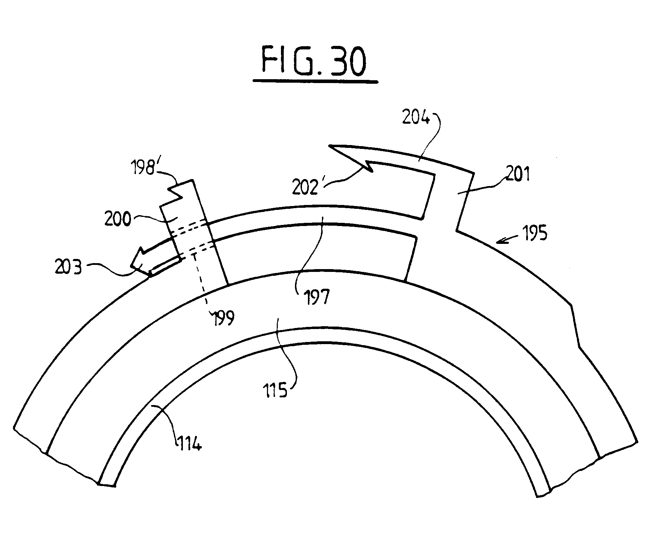

Dans la forme de réalisation illustrée sur les figures 28 et 29, l'accroissement de la dimension radiale du boudin d'étanchéité 123 est obtenu par un moyen mécanique du type "à coin". De façon plus précise, l'invention prévoit, dans cette réalisation, de constituer le corps 129', -qui joue le rôle du corps 129 de la réalisation selon les figures 16 à 18-, par une paroi cylindrique 190 qui peut être fixée au manchon 115 sans possibilité de mouvement relatif ou qui, en variante, est montée à rotation par rapport audit manchon par une nervure 130' logée dans une gorge 131' du manchon 115, ladite paroi étant solidaire d'une embase 191 sensiblement perpendiculaire à l'axe A du dispositif et qui présente une face externe, 192, inclinée sur la direction de cet axe. Sur l'embase 191, concentriquement à la paroi 190 et adjacente à celle-ci, une rondelle 193 à section droite triangulaire ménage avec le retour 140 du manchon 115 un logement de réception du boudin d'étanchéité 123. Ce dernier, à section droite quelque peu trapézoïdale, est avantageusement réalisé en un matériau élastique à base d'élastomère (d'une dureté de préférence comprise encre 20 et 40 Shore A) ou de plastomère et, dans ce dernier cas, un matériau mousse ou alvéolaire à peau intégrée est particulièrement bien approprié.

Quel que soit le matériau choisi, le boudin d'étanchéité 123 est un corps "plein" ou, en variante, un corps "toroïdal" enfermant une cavité 194 remplie d'un fluide incompressible, comme de l'eau.

Pour accroître la dimension radiale du boudin d'étanchéité 123 l'invention prévoit, dans cette forme de réalisation , un organe de manoeuvre 195 associé au porte-poches 110 et qui est constitué par un anneau fendu à section droite triangulaire rapporté sur l'embase 191 où il est maintenu dans la condition "ouverte" (montrée sur la figure 28) par coopération d'un crochet 196 prévu à l'extrémité d'une languette 197, solidaire d'une des extrémités de l'anneau, avec un ergot 198 ménagé à l'autre extrémité dudit anneau, la languette 197 étant propre à coulisser au travers d'une ouverture 199 de la patte d'extrémité 200 portant l'ergot 198. L'extrémité de l'anneau 195 opposée à celle portant la patte 200 est conformée suivant une patte 101 et la languette 197 porte, au voisinage de cette patte, une dent 202.

Pour la mise en oeuvre de l'appareillage, et après que le porte-poches ait été fixé au corps de l'utilisateur, une poche 120 est rapportée sur le porte-poches avec le nez de poche 121 en regard du bourrelet d'étanchéité 123 ou, en variante, est mise en place comme décrit en référence aux figures 17A et 17B ; l'anneau 195 étant dans la condition "ouverte" montrée sur la figure 28 (c'est-à-dire avec le crochet 196 en prise avec l'ergot 198), ledit anneau ne coopère pas avec la rondelle 193 et lorsque les pattes 200 et 201 sont rapprochées l'une de l'autre, la languette 197 coulisse dans le passage 199 et l'anneau 195 s'engage sous la rondelle 193 : le boudin d'étanchéité 123 immobilisé par le flasque 140 et la paroi cylindrique 190 se déforme alors suivant la direction de la flèche m, c'est-à-dire pour accroître sa dimension radiale et cela jusqu'à contact avec le nez de poche 121 réalisé en un matériau relativememt rigide pour former surface d'appui à étanchéité et blocage de la poche sur le porte-poches.

La condition d'assemblage est conservée aussi longtemps que la dent 202 de la languette 197 coopère avec l'ergot 198, pour maintenir l'organe 195 en condition "fermée", tandis que la libération de la dent 202 par rapport audit ergot ramène par effet élastique l'organe 195 dans la condition montrée sur la figure 28 en laquelle, -le boudin d'étanchéité 123 ayant lui aussi repris sa condition initiale-, la poche 120 peut être retirée du porte-poches 110.

Dans la réalisation montrée schématiquement sur la figure 30 une dent 202′ (analogue à la dent 202 de la réalisation selon les figures 28 et 29) est associée à la patte 201, cette dent étant propre à coopérer avec un ergot 198′ (analogue à l'ergot 198) prévu sur la patte 200. Dans cette réalisation, dont le fonctionnement est analogue à celui décrit ci-dessus en référence aux figures 28 et 29, la languette 197, à extrémité 203 en pointe de harpon, coulisse dans le passage 199 lors du rapprochement des pattes 200 et 201 que l'utilisateur peut serrer entre ses doigts jusqu'à coopération de la dent 202′ et de l'ergot 198′, tandis que la désolidarisation de la dent 202′ par rapport à l'ergot 198′, le plus simplement en soulevant l'extrémité de la languette 204 qui porte ladite dent, ramène par effet élastique l'organe 195 dans la condition montrée sur la figure 30.

Le fait pour l'organe de manoeuvre 195 de pouvoir tourner librement autour de l'axe A du dispositif, dans sa condition montrée sur les figures 28 ou 30, offre à l'utilisateur l'avantage de positionner au mieux, pour son confort et/ou pour leur manipulation, les pattes 200 et 201.

## Revendications

1. Appareillage pour stomie comportant un porte-poches (10, 110) destiné à être fixé autour d'une ouverture artificielle du corps d'un utilisateur à l'aide d'un patin muni d'un adhésif ou d'une gomme adhésive sensible à la pression ou par un moyen équivalent ainsi qu'une poche de recueil des fluides et/ou déchets corporels propre à être assemblée de manière amovible par un nez qu'elle présente au porte-poches présentant un manchon ou collet, caractérisé en ce que la fixation de la poche (40, 40', 60, 120) sur le porte-poches (10, 110) résulte de la déformation d'un moyen d'étanchéité (30, 20', 64, 122) dont on accroît la dimension radiale par manoeuvre d'un dispositif d'actionnement (20, 20', 66, 80, 100, 128, 195) approprié.

2. Appareillage selon la Revendication 1, caractérisé en ce que le dispositif d'actionnement est un anneau fendu (20, 80) auquel est associé le moyen d'étanchéité constitué par un joint d'étanchéité (30) en matériau élastique interposé entre le nez de poche (41) et l'anneau lorsque la poche est rapportée sur le porte-poches (10).

3. Appareillage selon la Revendication 2, caractérisé en ce que l'anneau fendu (80) est d'une pièce avec un moyen d'actionnement (83) constitué par un levier articulé sur l'anneau et qui commande un mécanisme du type "à genouillère" (85, 86).

4. Appareillage selon la Revendication 1, caractérisé en ce que le dispositif d'actionnement est constitué par deux demi-anneaux auxquels est associé le moyen d'étanchéité (30) en matériau élastique interposé entre le nez de poche (41) et les demi-anneaux lorsque la poche est rapportée sur le porte-poches.

5. Appareillage selon la Revendication 4, caractérisé en ce que chaque demi-anneau est d'une pièce avec un moyen d'actionnement constitué par un levier articulé sur lui et qui commande un mécanisme à genouillère.

6. Appareillage selon la Revendication 4, caractérisé en ce que les deux demi-anneaux (80ₐ, 80_{b}) sont réunis entre eux par un mécanisme "à genouillère" lui même constitué par deux bras (85, 86) articulés entre eux autour d'une charnière(87) et sur les demi-anneaux (80ₐ, 80_{b}), respectivement, par des charnières (88, 89) dont la première forme aussi l'articulation du levier (83).

7. Appareillage selon la Revendication 4, caractérisé en ce que les deux demi-anneaux (100ₐ et 100_{b}) sont réunis entre eux par deux mécanismes à genouillères constitués chacun par deux bras (95, 96, 95′, 96′) articulés entre eux autour d'une charnière (97, 97′) et sur les demi-anneaux (100ₐ, 100_{b}) par des charnières (99, 99′), les articulations des leviers (103, 103′) étant réalisées autour de deux axes charnières (98, 98′) solidaires de la collerette (15) par une pièce (104).

8. Appareillage selon la Revendication 2, caractérisé en ce que le joint d'étanchéité (30) est à section droite de forme trapézoïdale et enserre un retour (22) de l'anneau fendu, la déformation de ce dernier lors de la manoeuvre du moyen d'actionnement (50, 83) entraînant celle du joint d'étanchéité (30) jusqu'à application de celui-ci à étanchéité sur le nez de poche (41).

9. Appareillage selon l'une quelconque des Revendications précédentes, caractérisé en ce que le nez de poche (41, 61) est en une matière plastique suffisamment rigide pour encaisser l'effort de serrage développé par le joint d'étanchéité (30, 64) avec lequel il coopère par sa face interne (42, 62) à section de forme générale hyperbolique dans la condition d'assemblage de la poche (40, 60) et du porte-poches.

10. Appareillage selon l'une quelconque des Revendications précédentes, caractérisé en ce que pour le montage du dispositif d'actionnement (20, 20′, 66, 80) sur le porte-poches ce dernier présente une gorge (19, 19′, 70) limitée par un collet (17, 71), -qui entoure la stomie dans la condition d'utilisation de l'appareillage-, et par deux collerettes (15, 18 ; 15′, 47 ; 68, 67) sensiblement parallèles et perpendiculaires à l'axe (A) du collet, une première collerette (18, 67) coopérant au maintien du joint d'étanchéité (30, 64) et la seconde (15, 15′, 68) servant à la solidarisation du porte-poches et du patin (12), d'une part, ainsi qu'au guidage et/ou à la retenue du dispositif d'actionnement sur ledit porte-poches, d'autre-part.

11. Appareillage selon la Revendication 10, caractérisé en ce que la collerette (15, 15′) de guidage et/ou de retenue du dispositif d'actionnement est munie de lumières (26) dans lesquelles sont logés des tétons (25) du dispositif d'actionnement.

12. Appareillage selon la Revendication 1, caractérisé en ce que le moyen d'étanchéité est un anneau fendu (20′) conformé pour ménager sur sa périphérie des saillies (46) propres à coopérer avec des évidements ou une rainure (45) de forme conjuguée prévus sur le nez de poche (41′).

13. Appareillage selon la Revendication 1, caractérisé en ce que c'est un même organe élastiquement déformable (66) qui joue le rôle de joint d'étanchéité et de dispositif d'actionnement.

14. Appareillage selon la Revendication 13, caractérisé en ce que l'organe élastiquement déformable est une chambre torique gonflable et dégonflable et en ce que le moyen de gonflage (66′) de la chambre torique (66) est un dispositif du type seringue, une réserve de fluide gazeux sois pression comme une bombe portative d'air comprimé ou un dispositif hydraulique dans lequel le déplacement d'un levier permet de transférer dans et hors de ladite chambre (66) un volume donné de liquide.

15. Appareillage selon la Revendication 1, caractérisé en ce que le moyen d'étanchéité (122) dont on accroît la dimension radiale comprend un boudin d'étanchéité proprement dit (123) monté à déplacement radial par rapport au manchon (115) du porte-poches (110) et vers l'extérieur dudit manchon (115) lorsqu'on augmente le volume d'une chambre (128) adjacente audit boudin (123).

16. Appareillage selon la Revendication 15, caractérisé en ce que l'augmentation de volume de la chambre (128) adjacente au boudin d'étanchéité (123) résulte de l'introduction dans ladite chambre (128) d'une quantité de fluide supplémentaire par rapport à celle qu'elle contenait initialement, ladite introduction étant obtenue par des moyens mécaniques, comme une déformation locale à l'aide d'un levier ou analogue (161, 162 ... 170, 172) d'une capacité de volume donné (146) ou, en variante, par apport dans ladite chambre (128), directement ou indirectement, d'un fluide hydraulique ou pneumatique à partir d'une source de fluide sous pression, comme une seringue, une pompe, un réservoir de gaz comprimé, etc... (figures 23 à 26 et figure 27).

17. Appareillage selon la Revendication 15, caractérisé en ce que l'organe d'étanchéité (122) est un élément plein ou creux comportant des lèvres ou membranes (124, 125) de fixation sur un corps (129) solidaire du porte-poches (110) et qui ménage les moyens permettant de modifier le volume de la chambre (128) adjacente audit boudin d'étanchéité (123).

18. Appareillage selon la Revendication 17, caractérisé en ce que le corps (129) présente des canaux (145) débouchant dans une enceinte (146, 175) dont le volume peut être modifié et/ou dans laquelle peut être introduit un fluide sous pression.

19. Appareillage selon la Revendication 16, caractérisé en ce que la capacité (146) est limitée par une paroi (147) en matériau élastique sur laquelle est propre à agir un piston (160, 160a, 160b) dont le mouvement est commandé par l'actionnement d'un ou de levier(s) (161, 162 ; 170, 172).

20. Appareillage selon la Revendication 19, caractérisé en ce que la capacité (146) est enfermée dans un boîtier (148) solidaire du porte-poches (110) par des potences (153, 154), et en ce que lesdites potences présentent des crochets (157, 158) avec lesquels sont propres à coopérer des crochets conjugués du ou des levier(s) d'actionnement du piston (160, 160a, 160b) pour le maintien du ou desdits levier(s) dans leur position correspondant à celle d'assemblage de la poche (120) au porte-poches (110).

21. Appareillage selon l'une quelconque des Revendications 15 à 18, caractérisé en ce que la capacité (175) permettant d'alimenter la chambre de volume variable (128) adjacente au boudin d'étanchéité (123) à partir des canaux (145) peut être reliée à une source de fluide sois pression par un dispositif de valve (178, 178′) avec interposition d'un tube souple déformable (177, 186).

22. Appareillage selon la Revendication 21, caractérisé en ce que le dispositif de valve est relié à un clapet (185) lui même abouché à un tube du type soufflet (186) pour gonfler la capacité (175) par un moyen analogue à une pompe (figure 27).

23. Appareillage selon la Revendication 15, caractérisé en ce que les moyens pour accroître la dimension radiale du moyen d'étanchéité (122) comprennent un anneau fendu (195) et une rondelle (193) adjacente audit moyen d'étanchéité (122), l'anneau fendu (195) et la rondelle (193) agissant par un effet de "coin" pour la déformation radiale dudit moyen d'étanchéité (122).

24. Appareillage selon la Revendication 23, caractérisé en ce que l'anneau fendu (195) présente, à ses extrémités, des pattes (200, 201) d'actionnement au rapprochement desdites extrémités et des moyens de becs, crochets, ergots ou analogues, (196, 198, 202 ; 198′, 202′ 203) pour son maintien en position "ouverte" ou "fermée".

25. Appareillage selon la Revendication 24, caractérisé en ce que l'anneau fendu (195) comporte également une languette (197) solidaire d'une des pattes (201) et logée à coulissement dans un passage (199) de l'autre patte (200) pour le guidage l'une par rapport à l'autre desdites pattes lors de leurs mouvements de rapprochement et/ou d'éloignement.

26. Appareillage selon l'une quelconque des Revendications précédentes, caractérisé en ce que le moyen d'étanchéité et le corps qui le porte sont montés à rotation par rapport au manchon du porte-poches.

27. Appareillage selon l'une quelconque des Revendications précédentes, caractérisé en ce que le moyen d'étanchéité (30, 122) est en un matériau du type élastomère et/ou plastomère du type alvéolaire et/ou cellulaire à peau intégrée.

28. Appareillage selon la Revendication 27, caractérisé en ce que l'organe d'étanchéité (122) est un corps "plein" ou "creux" en un matériau élastomère d'une dureté comprise entre 20 et 40 Shore A.

29. Appareillage selon l'une quelconque des Revendications 15 à 28, caractérisé en ce que le moyen d'étanchéité (122) est maintenu à l'encontre d'un déplacement axial sur le porte-poches (110) par un flasque (140) et en ce que le diamètre interne (Φ₁) du nez de poche (121) est plus petit que le diamètre externe (Φ₂) dudit flasque mais plus grand que le diamètre externe (Φ₃) du moyen d'étanchéité (122).

30. Porte-poches propre à entrer dans la constitution d'un dispositif selon l'une quelconque des Revendications 1 à 29 comprenant un patin (11, 111) muni d'un adhésif ou d'une gomme adhésive sensible à la pression ou d'un moyen équivalent de fixation sur le corps d'un utilisateur avec un manchon (17, 115) intérieur au nez de poche (41, 121) dans la condition assemblée du dispositif, caractérisé en ce qu'il comporte autour du manchon (17, 115) un moyen d'étanchéité (30, 122) et un dispositif pour accroître la dimension radiale dudit moyen (30, 122) d'étanchéité pour fixer la poche sur le porte-poche.

## Claims

1. Stoma equipment comprising a bag-carrier (10, 110) for fixing around an artificial opening in the body of a user by means of a base plate provided with an adhesive or with a pressure-sensitive adhesive rubber or with any equivalent means, together with a bag for collecting body wastes and/or fluids and suitable for being removably assembled to a sleeve or collar of the bag carrier by means of a rim on the bag, the equipment being characterized in that the bag (40, 40', 60, 120) is fixed on the bag-carrier (10, 110) by deforming sealing means (30 20', 64, 122) whose radial size is increased by operating an appropriate actuator device (20, 20', 66, 80, 100, 128, 195).

2. Equipment according to claim 1, characterized in that the actuator device is a split ring (20, 80) which is associated with the sealing means constituted by a sealing ring (30) of resilient material interposed between the bag rim (41) and the split ring when the bag is applied to the bag-carrier (10).

3. Equipment according to claim 2, characterized in that the split ring (80) is integrally formed with actuator means (83) constituted by a lever hinged on the split ring and controlling a toggle type mechanism (85, 86).

4. Equipment according to claim 1, characterized in that the actuator device is constituted by two half-rings which are associated with the sealing means (30) made of resilient material interposed between the bag rim (41) and the half-rings when the bag is placed on the bag-carrier.

5. Equipment according to claim 4, characterized in that each half-ring is integral with actuator means constituted by a lever hinged thereto and controlling a toggle mechanism.

6. Equipment according to claim 4, characterized in that the two half-rings (80a, 80b) are connected to each other by a toggle mechanism itself constituted by two arms (85, 86) which are hinged to each other about a hinge (87) and which are hinged to respective ones of the half-rings (80a, 80b) by hinges (88, 89), the first of which also constitutes the fulcrum of a lever (83).

7. Equipment according to claim 4, characterized in that the two half-rings (100a, 100b) are interconnected by two toggle mechanisms, each constituted by a pair of arms (95, 96, 95', 96') hinged together about a hinge (97, 97') and hinged to the half-rings (100a, 100b) via respective hinges (99, 99'), fulcrums for levers (103, 103') being formed about two hinge axes (98, 98') fixed to the flange (15) by a part (104).

8. Apparatus according to claim 2, characterized in that the sealing ring (30) has a trapezium-shaped right cross-section and is engaged around a lip (22) on the split ring, deformation of the split ring during operation of the actuator means (50, 83) deforming the sealing ring (30) until it is applied in leakproof manner against the bag rim (41).

9. Equipment according to any preceding claim, characterized in that the bag rim (41, 61) is made of a plastic material that is rigid enough to accept the clamping force developed by the sealing ring (30, 64) with which its inside face (42, 62) of generally hyperbolic section co-operates when the bag (40, 60) is assembled to the bag-carrier.

10. Equipment according to any preceding claim, characterized in that to mount the actuator device (20, 20', 66, 80) on the bag-carrier, the bag-carrier has a groove (19, 19', 70) delimited by a collar (17, 71) which surrounds the stoma when the equipment is in use, and by two flanges (15, 18; 15', 47; 68, 67) which are substantially parallel to each other and perpendicular to the axis (A) of the collar, a first one of the flanges (18, 67) cooperating in retaining the sealing ring (30, 64) and a second one of the flanges (15, 15', 68) serving firstly to fix the bag-carrier to the base plate (12) and secondly to guide and/or retain the actuator device on said bag carrier.

11. Equipment according to claim 10, characterized in that the flange (15, 15') for guiding and/or retaining the actuator device is provided with slots (26) which receive studs (25) on the actuator device.

12. Equipment according to claim 1, characterized in that the sealing means is a split ring (20') shaped to have projections (46) at its periphery suitable for co-operating with hollows or a groove (45) of complementary shape provided in the bag rim (41').

13. Equipment according to claim 1, characterized in that the same elastically deformable member (66) acts both as the sealing ring and as the actuator device.

14. Equipment according to claim 13, characterized in that the elastically deformable member is an inflatable and deflatable toroidal chamber, and in that the inflation means (66') for inflating the toroidal chamber (66) is a syringe type device, a supply of gaseous fluid under pressure such as a portable compressed air cylinder, or a hydraulic device in which the displacement of a lever serves to transfer a given volume of liquid into or out from said chamber (66).

15. Apparatus according to claim 1, characterized in that the sealing means (122) whose radial size is increased comprises a sealing ring per se (123) mounted to move radially relative to the sleeve (115) of the bag-carrier (110) and away from said sleeve (115) when the volume of a chamber (128) adjacent to said sealing ring (123) is increased.

16. Equipment according to claim 15, characterized in that the volume of the chamber (128) adjacent to the sealing ring (123) is increased by inserting a quantity of fluid into said chamber (128) in addition to the fluid that it contained initially, said insertion being obtained by mechanical means such as locally deforming a space of given volume (146) by means of a lever or the like (161, 162, ..., 170, 172), or in a variant by directly or indirectly conveying a hydraulic or a pneumatic fluid into said chamber (128) from a source of fluid under pressure such as a syringe, a pump, a supply of compressed gas, etc. ... (Figures 23 to 26, and Figure 27).

17. Equipment according to claim 15, characterized in that the sealing means (122) is a solid or hollow component having lips or membranes (124, 125) for fixing to a body (129) integral with the bag-carrier (110) and including means enabling the volume of the chamber (128) adjacent to said sealing ring (123) to be modified.

18. Equipment according to claim 17, characterized in that the body (129) has channels (145) opening out into an enclosure (146, 175) whose volume can be modified and/or into which a fluid under pressure can be inserted.

19. Equipment according to claim 16, characterized in that the space (146) is delimited by a wall (147) of resilient material against which a piston (160, 160a, 160b) is disposed to act, displacement of the piston being controlled by actuating one or more levers (161, 162; 170, 172).

20. Equipment according to claim 19, characterized in that the space (146) is enclosed in a box (148) fixed to the bag-carrier (110) by brackets (153, 154), and in that said brackets have hooks (157, 158) suitable for co-operating with complementary hooks on the piston actuating lever(s) (160, 160a, 160b) to hold said lever(s) in a position corresponding to the bag (120) being assembled to the bag-carrier (110).

21. Equipment according to any one of claims 15 to 18, characterized in that the space (175) for feeding the variable volume chamber (128) adjacent to the sealing ring (123) via the channels (145) may be connected to a source of fluid under pressure by a valve device (178, 178') via a flexible deformable tube (177, 186).

22. Equipment according to claim 21, characterized in that the valve device is connected to a non-return valve (185) itself connected to a bellows type tube (186) for inflating the space (175) by means analogous to a pump (Figure 27).

23. Equipment according to claim 15, characterized in that the means for increasing the radial size of the sealing means (122) comprise a split ring (195) and a washer (193) adjacent to said sealing means (122), the split ring (195) and the washer (193) interacting by a wedge effect to deform said sealing means (122) radially.

24. Equipment according to claim 23, characterized in that the split ring (195) has actuator lugs (200, 201) at its ends for moving said ends towards each other and latches, hooks, catches, or the like (196, 198, 202; 198', 202', 203) for holding it in an open position or in a closed position.

25. Equipment according to claim 24, characterized in that the split ring (195) also includes a tongue (197) fixed to one of the lugs (201) and slidably received in a passage (199) through the other lug (200) to guide said lugs relative to each other while they move towards each other and/or while they move away from each other.

26. Equipment according to any preceding claim, characterized in that the sealing means and the body carrying the sealing means are mounted to rotate relative to the sleeve of the bag carrier.

27. Equipment according to any preceding claim, characterized in that the sealing means (30, 122) is made of an elastomer type material and/or of a cellular or foam type plastomer having an integral skin.

28. Equipment according to claim 27, characterized in that the sealing member (122) is a solid or a hollow body made of an elastomer material having hardness lying in the range 20 to 40 on the Shore A scale.

29. Equipment according to any one of claims 15 to 28, characterized in that the sealing means (122) is held on the bag-carrier (110) against axial displacement by means of flange (140), and in that the inside diameter (⌀1) of the bag rim (121) is smaller than the outside diameter (⌀2) of said flange but larger than the outside diameter (⌀3) of the sealing means (122).

30. A bag-carrier suitable for constituting part of equipment according to any one of claims 1 to 29 and comprising a base plate (11, 111) provided with an adhesive or with a pressure-sensitive adhesive rubber or with equivalent means for fixing to the body of a user, the bag-carrier having a sleeve (17, 115) for insertion inside the bag rim (41, 121) in the assembled condition of the equipment, the bag-carrier being characterized in that it includes sealing means (30, 122) around the sleeve (17, 115) and a device for increasing the radial size of said sealing means (30, 122) in order to fix the bag to the bag-carrier.

## Patentansprüche

1. Einrichtung für einen künstlichen Körperausgang, die folgendes aufweist: Einen Beutelhalter (10, 110), der dazu bestimmt ist, daß er um eine künstliche Öffnung des Körpers eines Benutzers herum mit Hilfe einer mit einem Kleber oder einem druckempfindlichen Klebergummi versehenen Unterlage oder mittels eines gleichwertigen Mittels befestigt wird, sowie einen Sammelbeutel für Fluida und/oder körperliche Ausscheidungen der durch einen Vorderteil auf abnehmbare Weise angebracht werden kann, den er für den eine Muffe oder einen Ansatz bildenden Beutelhalter bildet,
dadurch gekennzeichnet, daß sich die Befestigung des Beutels (40, 40', 60, 120) an dem Beutelhalter (10, 110) aus der Verformung eines Abdichtungsmittels (30, 20', 64, 122) ergibt, dessen radiale Abmessung durch Bedienen einer geeigneten Betätigungseinrichtung (20, 20', 66, 80, 100, 128, 195) vergrößert wird.

2. Einrichtung nach Anspruch 1, dadurch gekennzeichnet, daß die Betätigungseinrichtung ein geschlitzter Ring (20, 80) ist, mit welchem das Abdichtungsmittel verbunden wird, das aus einer Abdichtungsverbindung (30) aus einem elastischen Material besteht, welche zwischen den Beutelvorderteil (41) und den Ring gelegt wird, wenn der Beutel an den Beutelhalter (10) angesetzt wird.

3. Einrichtung nach Anspruch 2, dadurch gekennzeichnet, daß der geschlitzte Ring (80) mit einem Betätigungsmittel (83) einstückig ausgebildet ist, das aus einem Gelenkhebel auf dem Ring besteht, und das einen Mechanismus der Art mit "Kniegelenk" (85, 86) betätigt.

4. Einrichtung nach Anspruch 1 dadurch gekennzeichnet, daß die Betätigungseinrichtung aus zwei Halbringen besteht, mit welchen das Abdichtungsmittel (30) aus einem elastischen Material verbunden wird, welches zwischen den Beutelvorderteil (41) und die Halbringe gelegt wird, wenn der Beutel an den Beutelhalter angesetzt wird.

5. Einrichtung nach Anspruch 4 dadurch gekennzeichnet, daß jeder Halbring mit einem Betätigungsmittel einstückig ausgebildet ist, das aus einem Gelenkhebel auf demselben besteht und das einen Mechanismus mit Kniegelenk betätigt.

6. Einrichtung nach Anspruch 4, dadurch gekennzeichnet, daß die zwei Halbringe (80a, 80b) durch einen Mechanismus "mit Kniegelenk" miteinander verbunden sind, der seinerseits aus zwei Armen (85, 86) besteht, die aneinander um ein Drehgelenk (87) und auf den Halbringen (80a, 80b) jeweils über Drehgelenke (88, 89) angelenkt sind, bei welchen das erste auch das Gelenk des Hebels (83) bildet.

7. Einrichtung nach Anspruch 4, dadurch gekennzeichnet, daß die zwei Halbringe (100a und 100b) durch zwei Mechanismen mit Kniegelenk miteinander verbunden sind, bei welchen jeder aus zwei Armen (95, 96, 95', 96') besteht, die aneinander um ein Drehgelenk (97, 97') und auf den Halbringen (100ₐ, 100_{b}) über Drehgelenke (99, 99') angelenkt sind, wobei die Gelenke der Hebel (103, 103') um zwei Verbindungsachsen (98, 98') ausgeführt sind, die mit dem Kragen (15) über einen Teil (104) verbunden sind.

8. Einrichtung nach Anspruch 2, dadurch gekennzeichnet, daß die Abdichtungsverbindung (30) eine trapezförmige Querschnittsform aufweist und einen Rücksprung (22) des geschlitzten Ringes umschließt, wobei die Verformung dieses letzteren während der Bedienung des Betätigungsmittels (50, 83) jene der Abdichtungsverbindung (30) bis zum Ansetzen derselben an die Abdichtung auf dem Beutelvorderteil (41) mit sich bringt.

9. Einrichtung nach irgend einem der vorgehenden Ansprüche, dadurch gekennzeichnet, daß der Beutelvorderteil (41, 61) aus einem Kunststoff besteht, der hinreichend steif ist, um die von der Abdichtungsverbindung (30, 64) ausgeübte Spannkraft aufzunehmen, mit der er über seine Innenseite (42, 62) mit einem Querschnitt allgemeiner hyperbolischer Form während des Anbringens des Beutels (40, 60) und des Beutelhalters zusammenwirkt.

10. Einrichtung nach irgend einem der vorgehenden Ansprüche, dadurch gekennzeichnet, daß für das Anbringen der Betätigungseinrichtung (20, 20', 66, 80) an dem Beutelhalter dieser letztere einen Hals (19, 19', 70) bildet, der von folgendem begrenzt ist: einem Ansatz (17, 71), welcher in dem Zustand des Benutzens der Einrichtung den Eingang umgibt und zwei Kragen (15, 18; 15', 47; 68, 67), die im wesentlichen parallel und senkrecht zu der Achse (A) des Ansatzes liegen, wobei ein erster Kragen (18, 67) zum Festhalten der Abdichtungsverbindung (30, 64) zusammenwirkt und der zweite (15, 15', 68) einerseits zum Verbinden des Beutelhalters und der Auflage (12) sowie andererseits zum Führen und/oder zum Zurückhalten der Betätigungseinrichtung an dem Beutelhalter dient.

11. Einrichtung nach Anspruch 10, dadurch gekennzeichnet, daß der Kragen (15, 15') zum Führen und/oder zum Zurückhalten der Betätigungseinrichtung mit Öffnungen (26) versehen ist, in denen Zapfen (25) der Betätigungseinrichtung aufgenommen sind.

12. Einrichtung nach Anspruch 1, dadurch gekennzeichnet, daß das Abdichtungsmittel ein geschlitzter Ring (20') ist, der an seinem Umfang mit ausgesparten Vorsprüngen (46) ausgebildet ist, die mit Aussparungen oder einer Rille (45) mit einer zugeordneten Form zusammenwirken können, die an dem Beutelvorderteil (41') vorgesehen sind.

13. Einrichtung nach Anspruch 1, dadurch gekennzeichnet, daß es ein- und derselbe elastisch deformierbare Teil (66) ist, welcher die Rolle der Abdichtungsverbindung und der Betätigungseinrichtung spielt.

14. Einrichtung nach Anspruch 13, dadurch gekennzeichnet, daß der elastisch deformierbare Teil eine aufblasbare und ablaßbare torische Kammer ist und daß das Mittel (66') zum Aufblasen der torischen Kammer (66) eine Einrichtung der Art mit einer Spritze, eine Reserve für gasförmige Fluida unter Druck, wie eine tragbare Druckluftpatrone oder eine hydraulische Einrichtung ist, bei welcher die Verschiebung eines Hebels es ermöglicht, ein gegebenes Flussigkeitsvolumen in und aus der Kammer (66) zu überführen.

15. Einrichtung nach Anspruch 1, dadurch gekennzeichnet daß das Abdichtungsmittel (122), dessen radiale Abmessung vergrößert wird, genaugenommen eine Wulstdichtung (123) aufweist, die bezüglich der Muffe (115) des Beutelhalters (110) und nach außen von der Muffe (115) zum radialen Verschieben angebracht ist, wenn das Volumen einer zu dem Wulst (123) benachbarten Kammer (128) vergrößert wird.

16. Einrichtung nach Anspruch 15, dadurch gekennzeichnet, daß die Volumsvergrößerung der zu dem Dichtungswulst (123) benachbarten Kammer (128) das Einführen einer Menge zusätzlichen Fluids bezüglich jener, die sie ursprünglich enthielt, in die Kammer (128) ergibt, wobei die Einführung durch folgendes erhalten wird: mechanische Mittel, wie eine lokale Verformung einer gegebenen Volumskapazität (146) mit Hilfe eines Hebels oder ähnlichen (161, 162, ... 170, 172) oder als Variante unmittelbare oder mittelbare Zufuhr eines hydraulischen oder pneumatischen Fluids aus einer Fluidquelle unter Druck, wie einer Spritze, einer Pumpe, einem Reservoir für Druckgas, usw. in die Kammer (128) (Figuren 23 bis 26 und Figur 27).

17. Einrichtung nach Anspruch 15, dadurch gekennzeichnet, daß der Abdichtungsteil (122) ein volles oder ein hohles Element ist, das Lippen oder Membranen (124, 125) zum Befestigen an einem mit dem Beutelhalter (110) verbundenen Körper (129) aufweist und der es bewerkstelligt, daß die Mittel es ermöglichen, das Volumen der zu dem Abdichtungswulst (123) benachbarten Kammer (128) zu ändern.

18. Einrichtung nach Anspruch 17, dadurch gekennzeichnet, daß der Körper (129) in eine Umschließung (146, 175) mündende Kanäle (145) aufweist, deren Volumen geändert werden kann und/oder in die ein Fluid unter Druck geleitet werden kann.

19. Einrichtung nach Anspruch 16, dadurch gekennzeichnet, daß der Rauminhalt (146) durch eine Wand (147) aus einem elastischen Material begrenzt ist, auf die ein Kolben (160, 160a, 160b) wirken kann, dessen Bewegung durch das Betätigen eines Hebels oder von Hebeln (161, 162; 170, 172) angetrieben wird.

20. Einrichtung nach Anspruch 19, dadurch gekennzeichnet, daß der Rauminhalt (146) in einem mit dem Beutelhalter (110) durch Arme (153, 154) verbundenen Gehäuse (148) eingeschlossen ist und daß die Arme Haken (157, 158) aufweisen, mit welchen zugeordnete Haken des Hebels oder der Hebel zum Betätigen des Kolbens (160, 160a, 160b) zum Festhalten des Hebels oder der Hebel in ihrer Position zusammenwirken können, die jener zum Anbringen des Beutels (120) an dem Beutelhalter (110) entspricht.

21. Einrichtung nach einem der Ansprüche 15 bis 18, dadurch gekennzeichnet, daß der Rauminhalt (175), der es ermöglicht, die zu dem Dichtungswulst (123) benachbarte Kammer mit veränderlichem Volumen aus den Kanälen (145) zu versorgen, mit einer Quelle für Fluida unter Druck über eine Ventileinrichtung (178, 178') unter Einfügung einer deformierbaren biegsamen Röhre (177, 186) verbunden werden kann.

22. Einrichtung nach Anspruch 21, dadurch gekennzeichnet, daß die Ventileinrichtung mit einer Verschlußklappe (185) verbunden ist, die ihrerseits mit einer Röhre der Art, mit einem Faltenbalg (186) verbunden ist, um den Rauminhalt (175) durch ein einer Pumpe ähnliches Mittel aufzublasen (Figur 27).

23. Einrichtung nach Anspruch 15, dadurch gekennzeichnet, daß die Mittel zum Vergrößern der radialen Abmessung des Abdichtungsmittels (122) einen geschlitzten Ring (195) und einen zu dem Abdichtungsmittel (122) benachbarten Dichtungsring (193) aufweisen, wobei der geschlitzte Ring (195) und der Dichtungsring (193) durch einen Effekt eines "Keils" zum radialen Verformen des Abdichtungsmittels (122) wirken.

24. Einrichtung nach Anspruch 23, dadurch gekennzeichnet, daß der geschlitzte Ring (195) an seinen Enden folgendes aufweist: Pratzen (200, 201) zum Betätigen der Enden zur Annäherung und Mittel mit Nasen, Haken, Vorsprüngen oder ähnliches (196, 198, 202; 198', 202; 198', 202' 203) für sein Festhalten in einer Position "offen" oder "geschlossen".

25. Einrichtung nach Anspruch 24, dadurch gekennzeichnet, daß der geschlitzte Ring (195) gleichermaßen eine Zunge (197) aufweist, die mit einer der Pratzen (201) verbunden ist und verschiebbar in einem Durchgang (199) der anderen Pratze (200) aufgenommen ist, um die Pratzen während ihrer Bewegungen zum Annähern und/oder zum Entfernen aufeinanderbezogen zu führen.

26. Einrichtung nach irgend einem der vorgehenden Ansprüche, dadurch gekennzeichnet, daß das Abdichtungsmittel und der Körper, der dieses trägt bezüglich der Muffe des Beutelhalters drehbar angebracht sind.

27. Einrichtung nach einem der vorgehenden Ansprüche, dadurch gekennzeichnet, daß das Abdichtungsmittel (30, 122) aus einem Material der Elastomer- und/oder Plastomerart ist, welche der bienenzellenförmigen und/oder zellenförmigen Art mit integrierter Haut angehören.

28. Einrichtung nach Anspruch 27, dadurch gekennzeichnet, daß der Abdichtungsteil (122) ein "voller" oder "hohler" Körper aus einem Elastomermaterial ist, das eine Härte zwischen 20 und 40 Shore A aufweist.

29. Einrichtung nach irgendeinem der Ansprüche 15 bis 28, dadurch gekennzeichnet, daß das Abdichtungsmittel (122) gegen eine axiale Verschiebung an dem Beutelhalter (110) mittels eines Flansches (140) festgehalten wird, und daß der Innendurchmesser (⌀₁) des Beutelvorderteils (121) kleiner ist als der Außendurchmesser (⌀₂) des Flansches, jedoch größer als der Außendurchmesser (⌀₃) des Abdichtungsmittels (122).

30. Beutelhalter, der für den Aufbau einer Einrichtung nach irgendeinem der Ansprüche 1 bis 29 geeignet ist, mit einer Unterlage (11, 111), die mit folgendem versehen ist: Einem Kleber oder einem druckempfindlichen Klebergummi oder einem gleichwertigen Mittel zum Befestigen auf dem Körper des Benutzers, mit einer Muffe (17, 115), die sich im Inneren des Beutelvorderteils (41, 121) in dem angebrachten Zustand der Einrichtung befindet, dadurch gekennzeichnet, daß er um die Muffe (17, 115) herum ein Abdichtungsmittel (30, 122) und eine Einrichtung zum Vergrößern der radialen Abmessung des Abdichtungsmittels (30,122) aufweist, um den Beutel an der Beutelhalterung zu befestigen.
